(19)

Europäisches Patentamt

European Patent Office

Office européen des brevets

(11)  **EP 4 388 992 B1**

(12)  **EUROPEAN PATENT SPECIFICATION**

(45) Date of publication and mention
of the grant of the patent:
**27.05.2026  Bulletin 2026/22**

(21) Application number: **22877813.0**

(22) Date of filing: **23.08.2022**

(51) International Patent Classification (IPC):
*A61B 5/00* ^(2006.01)      *A61B 5/022* ^(2006.01)
*A61B 5/024* ^(2006.01)      *A61B 5/107* ^(2006.01)

(52) Cooperative Patent Classification (CPC):
**A61B 5/681; A61B 5/02233; A61B 5/02438;**
**A61B 5/02444; A61B 5/7475;** A61B 5/1075;
A61B 5/741; A61B 2562/0223

(86) International application number:
**PCT/CN2022/114113**

(87) International publication number:
**WO 2023/056790 (13.04.2023 Gazette 2023/15)**

(54) **AIRBAG DETECTION METHOD FOR WEARABLE DEVICE**

AIRBAGERKENNUNGSVERFAHREN FÜR WEARABLE-VORRICHTUNG

PROCÉDÉ DE DÉTECTION DE COUSSIN GONFLABLE POUR DISPOSITIF POUVANT ÊTRE
PORTÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **09.10.2021  CN 202111176128**

(43) Date of publication of application:
**26.06.2024  Bulletin 2024/26**

(73) Proprietor: **Huawei Technologies Co., Ltd.
Shenzhen, Guangdong 518129 (CN)**

(72) Inventor: **YUAN, Hao
Shenzhen, Guangdong 518129 (CN)**

(74) Representative: **Gill Jennings & Every LLP
The Broadgate Tower
20 Primrose Street
London EC2A 2ES (GB)**

(56) References cited:
**WO-A1-2012/059907     WO-A1-2023/025036
CN-A- 108 403 102     CN-A- 108 403 102
CN-A- 111 358 582     CN-A- 112 971 747
CN-A- 112 998 677     CN-A- 113 143 232
CN-A- 113 268 111     CN-A- 115 919 275
CN-U- 212 213 721     CN-U- 213 129 449**

US-A1- 2009 118 628     US-A1- 2020 085 319
US-A1- 2021 251 500

• **HUAWEI TECHNOLOGIES CO., LTD.:
"CN202122040209.9", 26 August 2021
(2021-08-26), pages 1 - 38, XP093147607,
Retrieved from the Internet <URL:NA>**
• **TEXAS INSTRUMENTS: "DRV5023 Digital-Switch
Hall Effect Sensor", 1 September 2016
(2016-09-01), Dallas, Texas 75265, USA, pages 1 -
32, XP093191773, Retrieved from the Internet
<URL:https://www.ti.com/lit/ds/symlink/drv5023.
pdf?ts=1722478935513>**
• **DARBAR RAJKUMAR ET AL: "Using Hall Effect
Sensors for 3D Space Text Entry on
Smartwatches", PROCEDIA COMPUTER
SCIENCE, ELSEVIER, AMSTERDAM, NL, vol. 84,
11 May 2016 (2016-05-11), pages 79 - 85,
XP029532996, ISSN: 1877-0509, DOI: 10.1016/
J.PROCS.2016.04.069**

EP 4 388 992 B1

• BUDIMAN AGUNG RAHMAT ET AL: "The Prototype of Smart Helmet with Safety Riding Notification for Motorcycle Rider", 2018 3RD INTERNATIONAL CONFERENCE ON INFORMATION TECHNOLOGY, INFORMATION SYSTEM AND ELECTRICAL ENGINEERING (ICITISEE), IEEE, 13 November 2018 (2018-11-13), pages 362 - 367, XP033554649, [retrieved on 20190522], DOI: 10.1109/ICITISEE.2018.8721027

**Description**

**TECHNICAL FIELD**

Embodiments of this application relate to wearable device technologies, and in particular, to a gasbag detection method and apparatus for a wearable device, and the wearable device.

**BACKGROUND**

With development of wearable devices, a current wearable device may provide a user with abundant function services. For example, if the wearable device is a watch, the watch may not only display time, but also provide the user with function services such as communication, entertainment, blood pressure measurement, and heart rate measurement.
When the user measures a blood pressure by using the watch, a gasbag disposed on the watch may be inflated by using the watch. If the inflated gasbag is small and cannot fully cover a wrist of the user, a blood pressure measurement result is inaccurate.
US 2020/085319 A1 discloses a wearable device capable of blood pressure measurement, comprising a wrist band assembly, a display unit provided on the wrist band assembly, and a detachable bladder provided on a backside of the wrist band assembly. CN 212213721 U discloses a watch-type blood pressure monitor with removable airbags, where the main body of the blood pressure monitor is provided with a bracket that can be installed with an airbag. CN 108403102 A discloses an electronic sphygmomanometer and a method for wearing an electronic sphygmomanometer. Darbar Rajkumar et al: "Using Hall Effect Sensors for 3D Space Text Entry on Smartwatches" (XP29532996A) discloses hall effect sensors based text entry mechanism that effectively uses the 3D space around the smartwatch for entering alphanumeric characters. US 2021/251500 A1 discloses a system and method for automatic determination of type of pneumatic cuff via an analysis of a pressure curve during inflation of the pneumatic cuff.

**SUMMARY**

Embodiments of this application provide a gasbag detection method and apparatus for a wearable device, and the wearable device, to improve accuracy of blood pressure detection. The invention is defined in the appended claims.
According to a first aspect, this application provides a gasbag detection method for a wearable device. An execution body of the method is the wearable device or a chip in the wearable device. The following uses an example in which the execution body of the method is the wearable device for description. In an embodiment, the wearable device may include but is not limited to a device that is used to be worn on a wrist of a user, like a watch or a band.
In the method, when a gasbag is mounted on the wearable device, the wearable device may detect, based on a wrist circumference of the user, whether the gasbag mounted on the wearable device matches the wrist circumference of the user; and if the gasbag mounted on the wearable device does not match the wrist circumference of the user, output first prompt information, where the first prompt information indicates to mount a gasbag that matches the wrist circumference of the user. After the user mounts, on the wearable device, the gasbag that matches the wrist circumference of the user, the user can accurately measure a blood pressure. In an embodiment, the first prompt information includes an identifier of the gasbag that matches the wrist circumference of the user.
If the gasbag mounted on the wearable device matches the wrist circumference of the user, the wearable device may inflate the gasbag by using an air pump in the wearable device, to detect the blood pressure of the user. The wearable device comprises a Hall effect sensor, an air pump, and a micro controller unit (MCU), the MCU being separately connected to the Hall effect sensor and the air pump. The Hall effect sensor comprises a positive pin, a negative pin, and a signal output pin. The air pump is disposed in the wearable device, and an air nozzle connected to the air pump is disposed on the outer surface of the wearable device. An air intake is disposed on the gasbag, and when the gasbag is mounted on the wearable device, the air nozzle is configured to communicate with the air intake of the gasbag.
In an embodiment, when the user needs to measure the blood pressure, the user may input the wrist circumference and an instruction for measuring the blood pressure in the wearable device. In an embodiment, when the user needs to measure blood pressure, if the wrist circumference is output, the user may input an instruction for measuring the blood pressure. In response to receiving an instruction indicating that the user inputs the wrist circumference, or receiving the instruction that is input by the user and that is for measuring the blood pressure, the wearable device may detect, based on the wrist circumference of the user, whether the gasbag mounted on the wearable device matches the wrist circumference of the user.
It should be understood that wrists of different users have different thicknesses, namely, different wrist circumference. For a user with a large wrist circumference, if a small-sized gasbag is used, the gasbag cannot fully cover a wrist of the user, and a blood pressure measurement result is inaccurate. For a user with a small wrist circumference, a large-sized gasbag can fully cover the wrist of the user, and a blood pressure measurement result is accurate. However, for the user with the small wrist circumference, a small-sized gasbag can fully cover the wrist of the user, and the small-sized gasbag is more convenient for use. Therefore, in this embodiment of this application, when the user performs blood pressure measurement by using the wearable device, it is important to recommend an appropriate gasbag to the user. This can improve user experience.

According to the first aspect, a premise of that the wearable device detects, based on the wrist circumference of the user, whether the gasbag mounted on the wearable device matches the wrist circumference of the user is that there is a gasbag mounted on the wearable device. Therefore, the wearable device further needs to detect whether the gasbag is mounted on the wearable device. Further, in response to detecting that the gasbag is mounted on the wearable device, the wearable device detects an identifier of the gasbag mounted on the wearable device, and detects, based on a mapping relationship between a wrist circumference and an identifier of a gasbag, whether the identifier of the gasbag mounted on the wearable device is the identifier of the gasbag mapped to the wrist circumference of the user. It should be understood that the wearable device pre-stores the mapping relationship between the wrist circumference and the identifier of the gasbag.

If the identifier of the gasbag mounted on the wearable device is not the identifier of the gasbag mapped to the wrist circumference of the user, the wearable device determines that the gasbag mounted on the wearable device does not match the wrist circumference of the user; or if the identifier of the gasbag mounted on the wearable device is the identifier of the gasbag mapped to the wrist circumference of the user, the wearable device determines that the gasbag mounted on the wearable device matches the wrist circumference of the user.

In an embodiment, the identifier of the gasbag may be, but is not limited to, information for distinguishing the gasbag, such as a type, a volume, and a number of the gasbag.

The following describes, from three aspects, a process in which the wearable device detects whether the gasbag is mounted on the wearable device, and detects the identifier of the gasbag mounted on the wearable device.

In a second manner, in response to sensing a positive magnetic pole, the positive pin outputs, through the signal output pin, a level greater than a second threshold; in response to sensing a negative magnetic pole, the negative pin outputs, through the signal output pin, the level greater than the second threshold; when the positive pin does not sense the positive magnetic pole, the positive pin outputs, through the signal output pin, a level less than a first threshold, where the second threshold is greater than the first threshold; and when the negative pin does not sense the negative magnetic pole, the negative pin outputs, through the signal output pin, the level less than the first threshold.

In other words, the Hall effect sensor may be configured to detect the positive magnetic pole or the negative magnetic pole.

In this manner, the wearable device includes at least two gasbags, magnetic poles are disposed on the gasbags of the at least two gasbags, and a different magnetic pole is disposed on each gasbag.

The wearable device may detect, based on the level output through the signal output pin, whether the gasbag is mounted on the wearable device. The wearable device may detect, based on the pin that outputs, through the signal output pin, the level greater than the second threshold, the identifier of the gasbag mounted on the wearable device.

For example, the at least two gasbags include a first gasbag and a second gasbag, the negative magnetic pole is disposed on the first gasbag, and the positive magnetic pole is disposed on the second gasbag. In this example, if the level output through the signal output pin is less than the first threshold, the wearable device determines that no gasbag is mounted on the wearable device. If the level output through the signal output pin is greater than the second threshold, the wearable device determines that the gasbag is mounted on the wearable device.

If the positive pin outputs, through the signal output pin, the level greater than the second threshold, the wearable device determines that the gasbag mounted on the wearable device is the second gasbag. If the negative pin outputs, through the signal output pin, the level greater than the second threshold, the wearable device determines that the gasbag mounted on the wearable device is the first gasbag.

In a second manner, the wearable device includes a differential pressure sensor and a gasbag watchband, the differential pressure sensor is configured to detect a differential pressure at the air nozzle of the air pump in the wearable device, the gasbag is mounted on the gasbag watchband, and when the gasbag watchband on which the gasbag is mounted is mounted on the wearable device, an air intake on the gasbag communicates with the air nozzle. Because volumes of gasbags with different identifiers are different, when the wearable device performs inflation by using the air pump, a change rate of the differential pressure of the gasbag is different. In this embodiment of this application, a change rate of the differential pressure at the air nozzle is used to represent the change rate of the differential pressure of the gasbag.

Specifically, the wearable device may control the air pump to blow air at a preset rate; obtain a change rate of the differential pressure within second preset duration based on the differential pressure at the air nozzle collected by the differential pressure sensor; further detect, based on the change rate of the differential pressure within the second preset duration, whether the gasbag is mounted on the wearable device; and detect, based on the change rate of the differential pressure within the second preset duration, the identifier of the gasbag mounted on the wearable device.

It should be understood that the wearable device further includes a non-gasbag watchband, and when the non-gasbag watchband is mounted on the wearable device, the non-gasbag watchband blocks the air nozzle of the air pump. Therefore, in the foregoing case, if the change rate of the differential pressure within the second preset duration is 0, or the change rate of the differential pressure within the second preset duration is greater than or equal to a first change rate threshold, the wearable device determines that no gasbag is mounted on the wearable device (in this case, no watchband is mounted on the wearable device, or the non-gasbag watchband is mounted on the wearable device).

If the change rate of the differential pressure within the second preset duration is less than the first change rate threshold, the wearable device determines that the gasbag is mounted on the wearable device. Further, in response to information that the change rate of the differential pressure within the second preset duration is less than the first change rate threshold, the wearable device detects, based on the change rate of the differential pressure within the second preset duration and a mapping relationship between a change rate of a differential pressure and an identifier of a gasbag, the identifier of the gasbag mounted on the wearable device.

In a possible implementation, the differential pressure sensor includes a first pressure sensor and a second pressure sensor, the first pressure sensor is configured to detect a first pressure at the air nozzle, the second pressure sensor is configured to detect a second pressure, the second pressure is an environmental pressure, and the differential pressure at the air nozzle is a difference between the first pressure and the second pressure.

In this embodiment of this application, the wearable device may obtain once the first pressure collected by the first pressure sensor at an interval of first preset duration, and obtain once the second pressure collected by the second pressure sensor at the interval of first preset duration. The wearable device obtains a first pressure average value based on first pressures collected within the second preset duration, obtains a second pressure average value based on second pressures collected within the second preset duration, and further obtains the change rate of the differential pressure within the second preset duration based on the first pressure average value and the second pressure average value.

In a third manner, the wearable device includes: the Hall effect sensor in the first manner, the differential pressure sensor, the gasbag watchband, and the at least two gasbags, the differential pressure sensor is configured to detect the differential pressure at the air nozzle of the air pump in the wearable device, the gasbag is mounted on the gasbag watchband, when the gasbag watchband on which the gasbag is mounted is mounted on the wearable device, the air intake on the gasbag communicates with the air nozzle, and magnetic poles disposed on at least two of the following are the same: the first gasbag in the at least two gasbags, the second gasbag in the at least two gasbags, and the non-gasbag watchband.

In this manner, the wearable device may detect, with reference to the Hall effect sensor and the change rate of the differential pressure of the gasbag, whether the gasbag is mounted on the wearable device, and detect the identifier of the gasbag mounted on the wearable device.

Specifically, the wearable device may control the air pump to blow air at the preset rate; obtain the change rate of the differential pressure within the second preset duration based on the differential pressure at the air nozzle collected by the differential pressure sensor; and detect, based on the level output through the signal output pin and the change rate of the differential pressure within the second preset duration, whether the gasbag is mounted on the wearable device; and the wearable device may detect, based on the pin that outputs, through the signal output pin, the level greater than the second threshold and the change rate of the differential pressure within the second preset duration, the identifier of the gasbag mounted on the wearable device.

For example, the negative magnetic pole is disposed on the first gasbag, the positive magnetic pole is disposed on the second gasbag, and the positive magnetic pole is disposed on the non-gasbag watchband. If the level output through the signal output pin is less than the first threshold, the wearable device determines that no gasbag is mounted on the wearable device. If the level output through the signal output pin is greater than the second threshold, and the negative pin outputs, through the signal output pin, the level greater than the second threshold, the wearable device determines that the gasbag mounted on the wearable device is the first gasbag. If the level output through the signal output pin is greater than the second threshold, and the positive pin outputs, through the signal output pin, the level greater than the second threshold, the wearable device detects, based on the change rate of the differential pressure within the second preset duration, that the second gasbag or the non-gasbag watchband is mounted on the wearable device.

When the non-gasbag watchband is mounted on the wearable device, the non-gasbag watchband blocks the air nozzle of the air pump. In this manner, if the change rate of the differential pressure within the second preset duration is greater than or equal to the first change rate threshold, the wearable device determines that the non-gasbag watchband is mounted on the wearable device. If the change rate of the differential pressure within the second preset duration is less than the first change rate threshold, the wearable device determines that the gasbag mounted on the wearable device is the second gasbag.

According to a second aspect, the disclosure also relates to a gasbag detection apparatus for a wearable device. The gasbag detection apparatus for the wearable device may be a wearable device, or a chip or a processor in the wearable device. The gasbag detection apparatus for the wearable device includes:

a detection module, configured to: when a gasbag is mounted on the wearable device, detect, based on a wrist circumference of a user, whether the gasbag mounted on the wearable device matches the wrist circumference of the user; and

an output module, configured to: if the gasbag mounted on the wearable device does not match the wrist circumference of the user, output first prompt information, where the first prompt information indicates to mount a gasbag that matches the wrist circumference of the user.

In a possible implementation, the detection module is further configured to detect whether the gasbag is mounted on the wearable device.

The detection module is specifically configured to: in response to detecting that the gasbag is mounted on the wearable device, detect an identifier of the gasbag mounted on the wearable device, and detect, based on a mapping relationship between a wrist circumference and an identifier of a gasbag, whether the identifier of the gasbag mounted on the wearable device is an identifier of a gasbag mapped to the wrist circumference of the user.

In a possible implementation, the detection module is specifically configured to: if the identifier of the gasbag mounted on the wearable device is not the identifier of the gasbag mapped to the wrist circumference of the user, determine that the gasbag mounted on the wearable device does not match the wrist circumference of the user; or if the identifier of the gasbag mounted on the wearable device is the identifier of the gasbag mapped to the wrist circumference of the user, determine that the gasbag mounted on the wearable device matches the wrist circumference of the user.

The wearable device includes a Hall effect sensor, and the Hall effect sensor includes a positive pin, a negative pin, and a signal output pin.

In response to sensing a positive magnetic pole, the positive pin outputs, through the signal output pin, a level greater than a second threshold; in response to sensing a negative magnetic pole, the negative pin outputs, through the signal output pin, the level greater than the second threshold; when the positive pin does not sense the positive magnetic pole, the positive pin outputs, through the signal output pin, a level less than a first threshold, where the second threshold is greater than the first threshold; and when the negative pin does not sense the negative magnetic pole, the negative pin outputs, through the signal output pin, the level less than the first threshold.

In a possible implementation, the wearable device includes at least two gasbags, magnetic poles are disposed on the gasbags of the at least two gasbags, and a different magnetic pole is disposed on each gasbag.

The detection module is specifically configured to: detect, based on the level output through the signal output pin, whether the gasbag is mounted on the wearable device; and detect, based on the pin that outputs, through the signal output pin, the level greater than the second threshold, the identifier of the gasbag mounted on the wearable device.

In a possible implementation, the at least two gasbags include a first gasbag and a second gasbag, the negative magnetic pole is disposed on the first gasbag, and the positive magnetic pole is disposed on the second gasbag.

The detection module is specifically configured to: if the level output through the signal output pin is less than the first threshold, determine that no gasbag is mounted on the wearable device; or if the level output through the signal output pin is greater than the second threshold, determine that the gasbag is mounted on the wearable device.

In a possible implementation, if the positive pin outputs, through the signal output pin, the level greater than the second threshold, it is determined that the gasbag mounted on the wearable device is the second gasbag; or if the negative pin outputs, through the signal output pin, the level greater than the second threshold, the detection module is specifically configured to determine that the gasbag mounted on the wearable device is the first gasbag.

In a possible implementation, the wearable device includes a differential pressure sensor and a gasbag watchband, the differential pressure sensor is configured to detect a differential pressure at an air nozzle of an air pump in the wearable device, the gasbag is mounted on the gasbag watchband, and when the gasbag watchband on which the gasbag is mounted is mounted on the wearable device, an air intake on the gasbag communicates with the air nozzle.

The detection module is specifically configured to: control the air pump to blow air at a preset rate; obtain a change rate of the differential pressure within second preset duration based on the differential pressure at the air nozzle collected by the differential pressure sensor; detect, based on the change rate of the differential pressure within the second preset duration, whether the gasbag is mounted on the wearable device; and detect, based on the change rate of the differential pressure within the second preset duration, the identifier of the gasbag mounted on the wearable device.

In a possible implementation, the wearable device further includes a non-gasbag watchband, and when the non-gasbag watchband is mounted on the wearable device, the non-gasbag watchband blocks the air nozzle of the air pump.

If the change rate of the differential pressure within the second preset duration is 0, or the change rate of the differential pressure within the second preset duration is greater than or equal to a first change rate threshold, the detection module is specifically configured to determine that no gasbag is mounted on the wearable device; or

if the change rate of the differential pressure within the second preset duration is less than the first change rate threshold, the detection module is specifically configured to determine that the gasbag is mounted on the wearable device.

In a possible implementation, the detection module is specifically configured to: in response to information that the change rate of the differential pressure within the second preset duration is less than the first change rate threshold, detect, based on the change rate of the differential pressure within the second preset duration and a mapping relationship between a change rate of a differential pressure and an identifier of a gasbag, the identifier of the gasbag mounted on the wearable device.

In a possible implementation, the wearable device includes: a differential pressure sensor, a gasbag watchband, and at least two gasbags, the differential pressure sensor is configured to detect a differential pressure at an air nozzle of an air pump in the wearable device, the gasbag is mounted on the gasbag watchband, when the gasbag watchband on which the gasbag is mounted is mounted on the wearable device, an air intake on the gasbag communicates with the air nozzle, and magnetic poles disposed on at least two of the following are the same: a first gasbag in the at least two gasbags, a second gasbag in the at least two gasbags, and a non-gasbag watchband.

The detection module is specifically configured to: control the air pump to blow air at a preset rate; obtain a change rate of the differential pressure within second preset duration based on the differential pressure at the air nozzle collected by the differential pressure sensor; detect, based on the level output through the signal output pin and the change rate of the differential pressure within the second preset duration, whether the gasbag is mounted on the wearable device; and detect, based on the pin that outputs, through the signal output pin, the level greater than the second threshold and the change rate of the differential pressure within the second preset duration, the identifier of the gasbag mounted on the wearable device.

In a possible implementation, the negative magnetic pole is disposed on the first gasbag, the positive magnetic pole is disposed on the second gasbag, and the positive magnetic pole is disposed on the non-gasbag watchband.

The detection module is specifically configured to: if the level output through the signal output pin is less than the first threshold, determine that no gasbag is mounted on the wearable device; if the level output through the signal output pin is greater than the second threshold, and the negative pin outputs, through the signal output pin, the level greater than the second threshold, determine that the gasbag mounted on the wearable device is the first gasbag; or if the level output through the signal output pin is greater than the second threshold, and the positive pin outputs, through the signal output pin, the level greater than the second threshold, detect, based on the change rate of the differential pressure within the second preset duration, that the second gasbag or the non-gasbag watchband is mounted on the wearable device.

In a possible implementation, when the non-gasbag watchband is mounted on the wearable device, the non-gasbag watchband blocks the air nozzle of the air pump.

The detection module is specifically configured to: if the change rate of the differential pressure within the second preset duration is greater than or equal to the first change rate threshold, determine that the non-gasbag watchband is mounted on the wearable device; or if the change rate of the differential pressure within the second preset duration is less than the first change rate threshold, determine that the gasbag mounted on the wearable device is the second gasbag.

In a possible implementation, the differential pressure sensor includes a first pressure sensor and a second pressure sensor, the first pressure sensor is configured to detect a first pressure at the air nozzle, the second pressure sensor is configured to detect a second pressure, the second pressure is an environmental pressure, and the differential pressure at the air nozzle is a difference between the first pressure and the second pressure.

The detection module is specifically configured to: obtain once the first pressure collected by the first pressure sensor at an interval of first preset duration; obtain once the second pressure collected by the second pressure sensor at the interval of first preset duration; obtain a first pressure average value based on first pressures collected within the second preset duration; obtain a second pressure average value based on second pressures collected within the second preset duration; and obtain the change rate of the differential pressure within the second preset duration based on the first pressure average value and the second pressure average value.

In a possible implementation, a sending and receiving module is configured to: receive an instruction indicating that the user inputs the wrist circumference, or receive an instruction that is input by the user and that is for measuring a blood pressure.

According to a third aspect, the disclosure also relates to a wearable device, including a Hall effect sensor. The Hall effect sensor includes a positive pin, a negative pin, and a signal output pin.

In response to sensing a positive magnetic pole, the positive pin outputs, through the signal output pin, a level greater than a second threshold; in response to sensing a negative magnetic pole, the negative pin outputs, through the signal output pin, the level greater than the second threshold; when the positive pin does not sense the positive magnetic pole, the positive pin outputs, through the signal output pin, a level less than a first threshold, where the second threshold is greater than the first threshold; and when the negative pin does not sense the negative magnetic pole, the negative pin outputs, through the signal output pin, the level less than the first threshold.

In a possible implementation, the wearable device includes at least two gasbags and a microprocessor unit MCU, and the MCU is connected to the Hall effect sensor; and the MCU is configured to detect the level output through the signal output pin.

In a possible implementation, magnetic poles are disposed on the gasbags of the at least two gasbags, and a different magnetic pole is disposed on each gasbag.

The MCU is further configured to: detect, based on the level output through the signal output pin, whether the gasbag is mounted on the wearable device; and detect, based on the pin that outputs, through the signal output pin, the level greater than the second threshold, an identifier of the gasbag mounted on the wearable device.

In a possible implementation, the wearable device further includes a differential pressure sensor and a gasbag watchband, the differential pressure sensor is configured to detect a differential pressure at an air nozzle of an air pump in the wearable device, the gasbag is mounted on the gasbag watchband, and when the gasbag watchband on which the gasbag is mounted is mounted on the wearable device, an air intake on the gasbag communicates with the air nozzle.

The MCU is further configured to: control the air pump to blow air at a preset rate; obtain a change rate of the differential pressure within second preset duration based on the differential pressure at the air nozzle collected by the differential pressure sensor; detect, based on the change rate of the differential pressure within the second preset duration, whether the gasbag is mounted on the wearable device; and detect, based on the change rate of the differential pressure within the second preset duration, the identifier of the gasbag mounted on the wearable device.

In a possible implementation, the wearable device further includes: a differential pressure sensor, a gasbag watchband, and the at least two gasbags, the differential pressure sensor is configured to detect a differential pressure at an air nozzle of an air pump in the wearable device, the gasbag is mounted on the gasbag watchband, when the gasbag watchband on which the gasbag is mounted is mounted on the wearable device, an air intake on the gasbag communicates with the air nozzle, and magnetic poles disposed on at least two of the following are the same: a first gasbag in the at least two gasbags, a second gasbag in the at least two gasbags, and a non-gasbag watchband.

The MCU is further configured to: control the air pump to blow air at a preset rate; obtain a change rate of the differential pressure within second preset duration based on the differential pressure at the air nozzle collected by the differential pressure sensor; detect, based on the level output through the signal output pin and the change rate of the differential pressure within the second preset duration, whether the gasbag is mounted on the wearable device; and detect, based on the pin that outputs, through the signal output pin, the level greater than the second threshold and the change rate of the differential pressure within the second preset duration, an identifier of the gasbag mounted on the wearable device.

In a possible implementation, the differential pressure sensor includes a first pressure sensor and a second pressure sensor, the first pressure sensor is disposed at the air nozzle, the first pressure sensor is configured to detect a first pressure at the air nozzle, the second pressure sensor is configured to detect a second pressure, the second pressure is an environmental pressure, and the differential pressure at the air nozzle is a difference between the first pressure and the second pressure.

In a possible implementation, the positive pin, the negative pin, and the air nozzle are disposed on an outer surface of the wearable device.

According to a fourth aspect, the disclosure also relates to a wearable device. The wearable device may include a processor and a memory. The memory is configured to store computer-executable program code, where the program code includes instructions. When the processor executes the instructions, the instructions enable the wearable device to perform the method in the first aspect.

According to a fifth aspect, the disclosure also relates to a wearable device. The wearable device may be the gasbag detection apparatus for the wearable device in the second aspect or the wearable device in the first aspect. The wearable device may include a unit, module, or circuit configured to perform the method in the first aspect.

According to a sixth aspect, the disclosure also relates to a computer program product including instructions. When the instructions are run on a computer, the computer is enabled to perform the method in the first aspect.

According to a seventh aspect, the disclosure also relates to a computer-readable storage medium. The computer-readable storage medium stores instructions. When the instructions are run on a computer, the computer is enabled to perform the method in the first aspect.

For beneficial effects of the possible implementations of the second aspect to the seventh aspect, refer to beneficial effects brought by the first aspect. Details are not described herein again.

The disclosure generally relates to a gasbag detection method and apparatus for a wearable device, and a wearable device. The method includes: when a gasbag is mounted on the wearable device, detecting, based on a wrist circumference of a user, whether the gasbag mounted on the wearable device matches the wrist circumference of the user; and if the gasbag mounted on the wearable device does not match the wrist circumference of the user, outputting first prompt information, where the first prompt information indicates to mount a gasbag that matches the wrist circumference of the user. In this embodiment of this application, the wearable device may detect, based on the wrist circumference of the user, whether the gasbag mounted on the wearable device matches the wrist circumference of the user. When the gasbag mounted on the wearable device does not match the wrist circumference, the user may be prompted to mount the gasbag that matches the wrist circumference of the user, to improve accuracy of blood pressure measurement.

## BRIEF DESCRIPTION OF DRAWINGS

FIG. 1 is a schematic diagram of a structure of a wearable device according to an embodiment of this application;

FIG. 2 is a schematic diagram of another structure of a wearable device according to an embodiment of this application;

FIG. 3 is a schematic diagram of detecting a gasbag by using a Hall effect sensor according to an embodiment of this application;

FIG. 4 is a schematic flowchart of an embodiment of a gasbag detection method for a wearable device according to an embodiment of this application;

FIG. 5 is a schematic diagram of an interface according to an embodiment of this application;

FIG. 6 is a schematic diagram of another interface according to an embodiment of this application;

FIG. 7 is a schematic diagram of another structure of a wearable device according to an embodiment of this application;

FIG. 8 is a schematic flowchart of another embodiment of a gasbag detection method for a wearable device according to an embodiment of this application; and

FIG. 9 is a schematic diagram of a structure of a gasbag detection apparatus for a wearable device according to an embodiment of this application.

## DESCRIPTION OF EMBODIMENTS

A wearable device in embodiments of this application may be, but is not limited to, a device that can be worn on a wrist of a user, like a watch or a band. The wearable device in embodiments of this application is configured to be worn on the wrist of the user, and a gasbag may be disposed on a side that is in contact with the wrist of the user. The gasbag is configured to measure a blood pressure of the user. It should be understood that the gasbag disposed on the wearable device in embodiments of this application is a detachable gasbag, and the wearable device can support gasbags of a plurality of types. Gasbags of different types have different sizes (which may be understood as volumes). In this way, fully inflated gasbags of different types can cover different areas of the wrist.

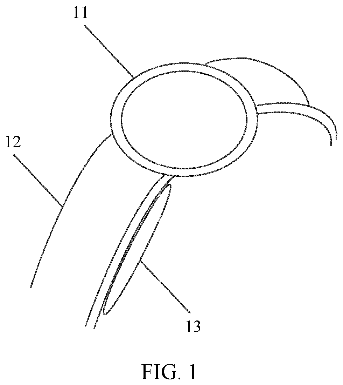

FIG. 1 is a schematic diagram of a structure of a wearable device according to an embodiment of this application. In FIG. 1, an example in which the wearable device is a watch is used for description. As shown in FIG. 1, the watch includes a watch head 11, a watchband 12, and a gasbag 13 disposed on the watchband 12. The watchband 12 may be mounted on the watch head 11, and the watchband 11 is detachable. The gasbag 13 may be mounted on the watchband, and the gasbag 13 is detachable. In other words, a user may mount the gasbag 13 on the watchband 12, or may remove the gasbag 13 from the watchband 12. The watch may support a plurality of types of gasbags 13. In other words, the user may remove one type of gasbag from the watchband 12, and mount another type of gasbag on the watchband 12. In FIG. 1, one gasbag is used as an example for description. It should be understood that the gasbag 13 in FIG. 1 is a gasbag that is not inflated.

In an embodiment, the gasbag 13 may be attached to the watchband 12. For example, a velcro is disposed on the gasbag 13 and the watchband 12, and the gasbag 13 and the watchband 12 may be attached together by using the velcro. Alternatively, in an embodiment, the gasbag 13 may be clamped on the watchband 12. For example, a through hole is disposed in the watchband 12, a buckle is disposed on the gasbag 13, and the buckle may be clamped in the through hole, so that the gasbag 13 is clamped on the watchband 12. In an embodiment, the velcro, the through hole, and the like that are disposed on/in the wearable device are gasbag detachment components. A manner in which the gasbag 13 is disposed on the watchband 12 is not limited in embodiments of this application, that is, the gasbag detachment component disposed on the wearable device is not limited. The two manners are examples for description.

It should be understood that the gasbag 13 is located on a side that is in contact with a wrist of the user. When the user wears the watch on the wrist, the gasbag 13 is in contact with the wrist of the user. When the user uses the watch to measure a blood pressure, the watch may be used to inflate the gasbag 13, to detect the blood pressure of the user. A principle in which the watch is used to inflate the gasbag 13 to detect the blood pressure of the user is not described herein. For details, refer to related descriptions in the conventional technology.

It should be understood that the watchband 12 on which the gasbag 13 is disposed may be referred to as a gasbag watchband. In an embodiment, a common watchband (the common watchband is a watchband on which the gasbag 13 cannot be mounted) may alternatively be mounted on the watch. In the following embodiments, the common watchband is referred to as a non-gasbag watchband.

Gasbags of different types have different sizes, and fully inflated gasbags can cover different areas of the wrist of the user. It may be figured out that a larger volume of the gasbag indicates a larger covered area of the wrist of the user, and a more accurate blood pressure measurement result; and a smaller volume of the gasbag indicates more convenient use. Wrists of different users have different thicknesses, namely, different wrist circumferences. Therefore, if a blood pressure of a user needs to be accurately measured, different users need different gasbags, and a result of measuring a blood pressure by using a gasbag of an incorrect type is inaccurate.

For example, for a user with a large wrist circumference, if a small-sized gasbag is used, the gasbag cannot fully cover the wrist of the user, and a blood pressure measurement result is inaccurate. For a user with a small wrist circumference, a large-sized gasbag can fully cover the wrist of the user, and a blood pressure measurement result is accurate. However, for the user with the small wrist circumference, a small-sized gasbag can fully cover the wrist of the user, and the small-sized gasbag is more convenient for use. Therefore, when the user performs blood pressure measurement by using the wearable device, it is important to recommend an appropriate gasbag to the user. This can improve user experience.

In this way, based on the foregoing problem, embodiments of this application provide a gasbag detection method for a wearable device. For the wearable device that supports a plurality of types of gasbags, when a user uses the wearable device to measure a blood pressure, the wearable device may first detect whether a gasbag currently mounted on the wearable device matches a wrist circumference of the user. If the gasbag matches the wrist circumference of the user, the wearable device may directly measure the blood pressure of the user, and accuracy of a blood pressure measurement result is high. If the gasbag does not match the wrist circumference of the user, the wearable device may prompt the user to replace the gasbag with a gasbag that matches the wrist circumference of the user, to improve accuracy of the blood pressure measurement result.

Before the gasbag detection method for a wearable device provided in embodiments of this application is described, a structure of the wearable device provided in embodiments of this application is first described.

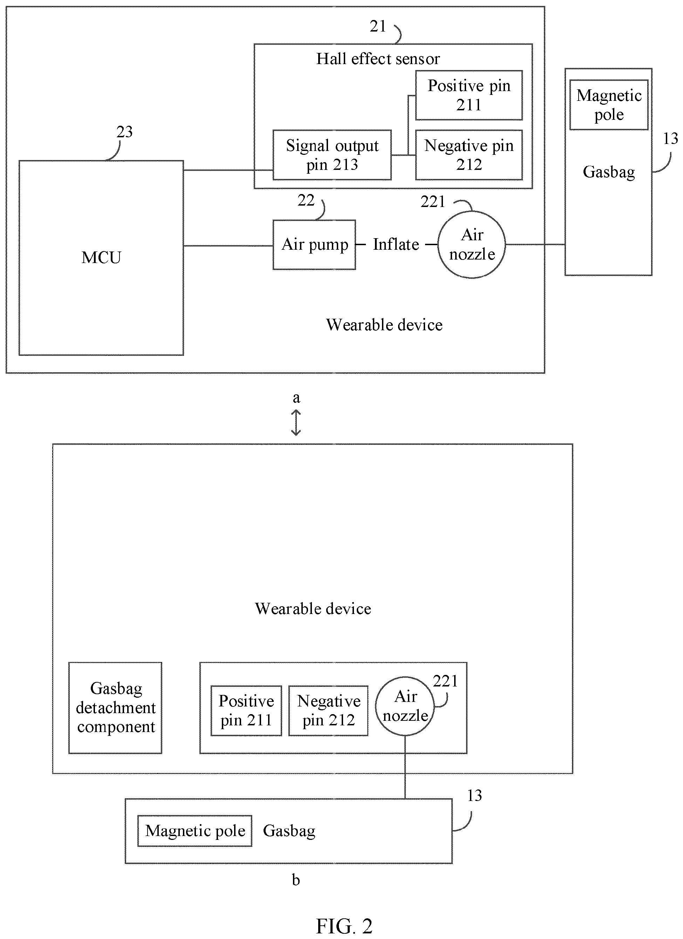

FIG. 2 is a schematic diagram of another structure of a wearable device according to an embodiment of this application. As shown in a in FIG. 2, the wearable device includes a Hall effect sensor 21, an air pump 22, and a micro controller unit (micro controller unit, MCU) 23. The MCU 23 is separately connected to the Hall effect sensor 21 and the air pump 22. In FIG. 2, an example in which the wearable device is a watch is used for description. In an embodiment, the Hall effect sensor 21, the air pump 22, and the MCU 23 are all disposed in a watch head 11 of the watch.

The Hall effect sensor 21 includes three pins: a positive (S pole) pin 211, a negative (N pole) pin 212, and a signal output pin 213. In an embodiment, the positive pin 211 and the negative pin 212 on the Hall effect sensor 21 are disposed on an outer surface of the watch head 11, and the signal output pin 213 on the Hall effect sensor 21 is disposed in the watch head 11. The air pump 22 is disposed in the watch head 11, and an air nozzle 221 connected to the air pump 22 is disposed on the outer surface of the watch head 11. It should be understood that, in embodiments of this application, locations at which the positive pin 211, the negative pin 212, and the air nozzle 221 are disposed on the outer surface of the watch head 11 are not limited. In b in FIG. 2, an example in which the positive pin 211, the negative pin 212, and the air nozzle 221 are disposed in parallel on the outer surface of the watch head 11 is used for description. It should be understood that a in FIG. 2 is a schematic diagram of a connection structure of the wearable device, and b in FIG. 2 is a schematic diagram of disposing the positive pin 211, the negative pin 212, and the air nozzle 221 on the outer surface of the wearable device.

It should be understood that, in an embodiment, a gasbag detachment component is disposed on the outer surface of the watch head, and a gasbag 13 may be mounted on the wearable device by using the gasbag detachment component. For the gasbag detachment component, refer to the related descriptions.

An air intake is disposed on the gasbag 13. When the gasbag 13 is mounted on the wearable device, the air nozzle 221 communicates with the air intake of the gasbag 13. When a user measures a blood pressure by using the wearable device, the MCU 23 may control the air pump 22 to blow air through the air nozzle 221, to inflate the gasbag 13. In an embodiment, the MCU 23 may send a pulse width modulation (pulse width modulation, PWM) wave to the air pump, to control the air pump 22 to blow air through the air nozzle 221.

In this embodiment, to cooperate with the positive pin 211 and the negative pin 212 in the wearable device, magnetic poles, for example, a positive magnetic pole (referred to as a positive pole for short below), or a negative magnetic pole (referred to as a negative pole for short below), may be disposed on gasbags of different types, or no positive or negative pole is disposed, to distinguish gasbags of different types (for a specific distinguishing manner, refer to the following related descriptions). It should be understood that a type represents different gasbags. Different types of gasbags may be understood as gasbags with different identifiers, and the identifiers may include but are not limited to a type, a volume, or a number. In the following embodiment, an example in which the identifier is a type is used for description.

It should be understood that the positive pin 211 on the Hall effect sensor 21 can sense the positive pole (S pole), and the negative pin 212 can sense the negative pole (N pole). When the magnetic pole approaches to the positive pin 211 or the negative pin 212, the positive pin 211 or the negative pin 212 can sense the magnetic pole, and the positive pin 211 or the negative pin 212 outputs a high level or a low level through the signal output pin 213 (the high level is used as an example for description below). Correspondingly, the positive pin 211 and the negative pin 212 may output a low level when the magnetic pole is not sensed. For example, when the positive pin 211 senses the positive pole (S pole), the positive pin 211 outputs a high level through the signal output pin 213; and when the negative pin 212 senses the negative pole (N pole), the negative pin 212 outputs a high level through the signal output pin 213. It should be understood that the low level may be understood as a level less than a first threshold, the high level may be understood as a level greater than a second threshold, and the second threshold is greater than the first threshold.

For example, in an embodiment, a first gasbag and a second gasbag in this embodiment of this application are used to represent gasbags of different types. A volume of the first gasbag is less than that of the second gasbag. For example, a negative pole is disposed on the first gasbag, a positive pole is disposed on the second gasbag, and no magnetic pole (a positive pole or a negative pole) is disposed on a non-gasbag watchband.

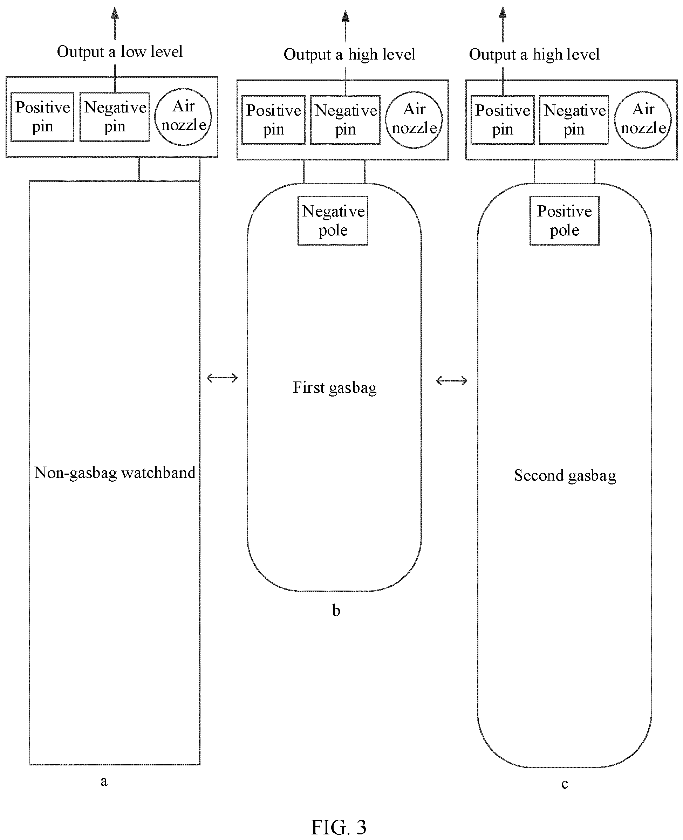

When no non-gasbag watchband is mounted on the watch head, or only a gasbag watchband is mounted on the watch head but no gasbag is mounted, the positive pin 211 and the negative pin 212 on the Hall effect sensor 21 do not sense approaching of the magnetic pole. Therefore, the positive pin 211 and the negative pin 212 may output a low level through the signal output pin 213. As shown in a in FIG. 3, when the user mounts the non-gasbag watchband on the watch head, because no magnetic pole is disposed on the non-gasbag watchband, the positive pin 211 and the negative pin 212 on the Hall effect sensor 21 do not sense approaching of the magnetic pole. Therefore, the positive pin 211 and the negative pin 212 may output a low level through the signal output pin 213. In a in FIG. 3, an example in which the negative pin 212 outputs a low level is used for description.

As shown in b in FIG. 3, when the user mounts the first gasbag on the gasbag watchband, and mounts the gasbag watchband on the watch head 11, the negative pole of the first gasbag is in contact with the negative pin 212 on the Hall effect sensor 21, and the negative pin 212 senses the negative pole. Therefore, a high level may be output through the signal output pin 213. As shown in c in FIG. 3, when the user mounts the second gasbag on the gasbag watchband, and mounts the gasbag watchband on the watch head 11, the positive pole of the second gasbag is in contact with the positive pin 211 on the Hall effect sensor 21, and the positive pin 211 senses the positive pole. Therefore, the positive pin 211 may output a high level through the signal output pin 213.

As shown in a in FIG. 2, the MCU 23 is connected to the Hall effect sensor 21, and the MCU 23 may detect the level output through the signal output pin 213 on the Hall effect sensor 21. In this way, the MCU 23 may detect, by detecting the level output through the signal output pin 213 on the Hall effect sensor 21, whether the gasbag is mounted on the wearable device, and detect, based on the positive pin or the negative pin that outputs, through the signal output pin, the level greater than the second threshold, an identifier of the gasbag mounted on the wearable device. In other words, the MCU 23 may detect, by detecting the level (a high level or a low level) output through the signal output pin 213 on the Hall effect sensor 21, whether the gasbag is mounted on the watch, and the MCU 23 may further detect a type of the gasbag by detecting the pin (the positive pin 211 or the negative pin 212) that outputs a high level.

For example, if the MCU 23 detects that a low level is output through the signal output pin 213 on the Hall effect sensor 21, it may be determined that no gasbag is mounted on the watch. When the MCU 23 detects that a high level is output through the signal output pin 213 on the Hall effect sensor 21, it may be determined that the gasbag is mounted on the watch. If the pin that outputs the high level is the positive pin 211, it may be determined that the gasbag mounted on the watch is the second gasbag. If the pin that outputs the high level is the negative pin 212, it may be determined that the gasbag mounted on the watch is the first gasbag.

It should be understood that, a positive pole may be disposed on the first gasbag, and a negative pole may be disposed on the second gasbag. The foregoing is an example for description. The magnetic pole disposed on the first gasbag is different from the magnetic pole disposed on the second gasbag, so that the user can distinguish between the first gasbag and the second gasbag.

Based on FIG. 2 and FIG. 3, the following describes, with reference to a specific embodiment, the gasbag detection method for a wearable device provided in embodiments of this application. The following several embodiments may be combined with each other, and a same or similar concept or process may not be described repeatedly in some embodiments.

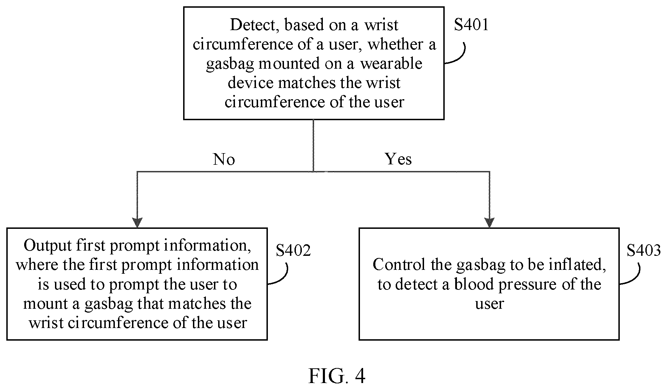

FIG. 4 is a schematic flowchart of an embodiment of a gasbag detection method for a wearable device according to an embodiment of this application. As shown in FIG. 4, the gasbag detection method for a wearable device provided in embodiments of this application may include the following steps.

S401: The wearable device detects, based on a wrist circumference of a user, whether a gasbag mounted on the wearable device matches the wrist circumference of the user. If the gasbag mounted on the wearable device does not match the wrist circumference of the user, S402 is performed; or if the gasbag mounted on the wearable device matches the wrist circumference of the user, S403 is performed.

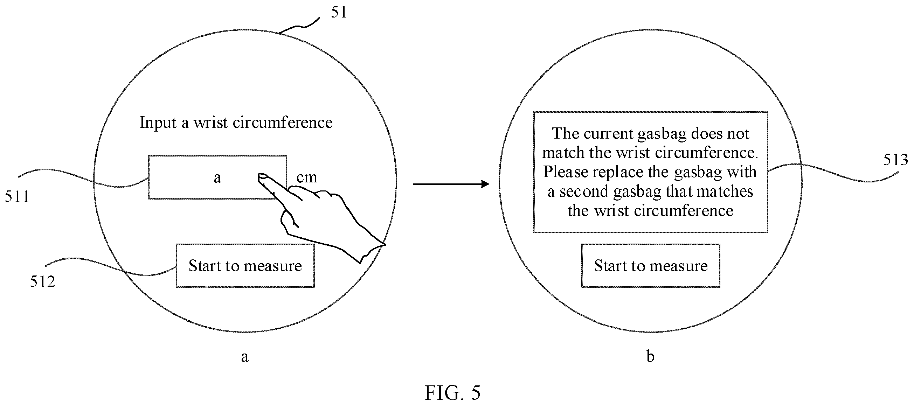

The wearable device provides a blood pressure measurement service for the user. When the user measures a blood pressure by using the wearable device, the user may input the wrist circumference of the user on an interface displayed on the wearable device or on an interface displayed on an electronic device (like a mobile phone or a tablet) bound to the wearable device. FIG. 5 is a schematic diagram of an interface according to an embodiment of this application. In FIG. 5, an example in which the user inputs the wrist circumference on the interface displayed on the wearable device is used for description. As shown in a in FIG. 5, an interface 51 of the wearable device displays an input box 511 and a measurement control 512 of the wrist circumference of the user.

When the user uses a blood pressure measurement function of the wearable device, that is, when the user has not input the wrist circumference, the wearable device may further output prompt information to prompt the user how to measure the wrist circumference. Details are not described herein. For details, refer to conventional related descriptions. The user may input the wrist circumference in the input box. It should be understood that FIG. 5 is an example in which the user inputs the wrist circumference on the interface displayed on the wearable device. The user may alternatively input the wrist circumference of the user to the wearable device in a voice or another manner.

In an embodiment, in response to detecting that the user inputs the wrist circumference in the input box 511, that is, detecting an instruction indicating that the user inputs the wrist circumference, the wearable device may detect, based on the wrist circumference of the user, whether the gasbag mounted on the wearable device matches the wrist circumference. In this embodiment, S401 may be replaced with S401A. S401A: In response to detecting the instruction indicating that the user inputs the wrist circumference, the wearable device detects, based on the wrist circumference of the user, whether the gasbag mounted on the wearable device matches the wrist circumference. If the gasbag mounted on the wearable device does not match the wrist circumference of the user, S402 is performed; or if the gasbag mounted on the wearable device matches the wrist circumference of the user, S403 is performed.

The wearable device may detect a type of the gasbag mounted on the wearable device, to detect whether the type of the gasbag matches the wrist circumference of the user. A manner in which the wearable device detects the type of the gasbag mounted on the wearable device may be as follows: The wearable device may detect, by detecting a level (a high level or a low level) output by the Hall effect sensor, whether the gasbag is mounted on the watch, and may further detect the type of the gasbag by detecting a pin that outputs a high level. For details, refer to the related descriptions in FIG. 2 and FIG. 3. In this embodiment, different magnetic poles are disposed on gasbags mounted on the wearable device. For example, a negative pole is disposed on a first gasbag, and a positive pole is disposed on a second gasbag.

In an embodiment, the wearable device may store a mapping relationship between a wrist circumference and a type of a gasbag, and the mapping relationship represents a type of a gasbag that matches each wrist circumference. For example, the mapping relationship may be shown in Table 1:

**Table 1**

| Wrist circumference (cm) | Type of the gasbag |
|---|---|
| a to b | First gasbag |
| b to c | Second gasbag |

After obtaining the type of the gasbag mounted on the wearable device, the wearable device may detect, based on the mapping relationship between the wrist circumference and the type of the gasbag, whether the gasbag mounted on the wearable device is the gasbag mapped to the wrist circumference. If the gasbag mounted on the wearable device is the gasbag mapped to the wrist circumference, it is determined that the gasbag mounted on the wearable device matches the wrist circumference, or if the gasbag mounted on the wearable device is not the gasbag mapped to the wrist circumference, it is determined that the gasbag mounted on the wearable device does not match the wrist circumference.

For example, if the wrist circumference of the user falls within a range from a cm to b cm, and the wearable device detects that the gasbag mounted on the wearable device is the second gasbag, or no gasbag is mounted on the wearable device, the wearable device may determine that the gasbag mounted on the wearable device does not match the wrist circumference. If the wearable device detects that the gasbag mounted on the wearable device is the first gasbag, the wearable device may determine that the gasbag mounted on the wearable device matches the wrist circumference.

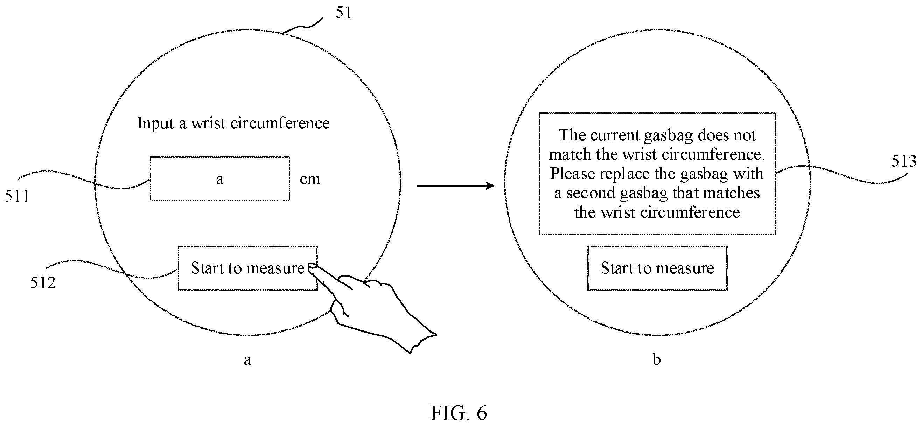

In an embodiment, when the user has used the blood pressure measurement function, that is, the user has input the wrist circumference in the input box 511, in this embodiment, as shown in a in FIG. 6, the user may directly operate the measurement control 512 without inputting the wrist circumference, to trigger the wearable device to start to measure the blood pressure of the user. In response to detecting that the user operates the measurement control 512, that is, detecting that the user inputs an instruction for measuring the blood pressure, the wearable device may detect, based on the wrist circumference of the user, whether the gasbag mounted on the wearable device matches the wrist circumference. Specifically, the wearable device may detect, based on a wrist circumference that is previously input by the user, whether the gasbag mounted on the wearable device matches the wrist circumference.

Correspondingly, in this embodiment, S401 may be replaced with S401B. S401B: In response to detecting that the user inputs the instruction for measuring the blood pressure, the wearable device detects, based on the wrist circumference of the user, whether the gasbag mounted on the wearable device matches the wrist circumference. If the gasbag mounted on the wearable device does not match the wrist circumference of the user, S402 is performed; or if the gasbag mounted on the wearable device matches the wrist circumference of the user, S403 is performed.

The wearable device detects, based on the wrist circumference of the user, whether the gasbag mounted on the wearable device matches the wrist circumference, which may refer to the related descriptions.

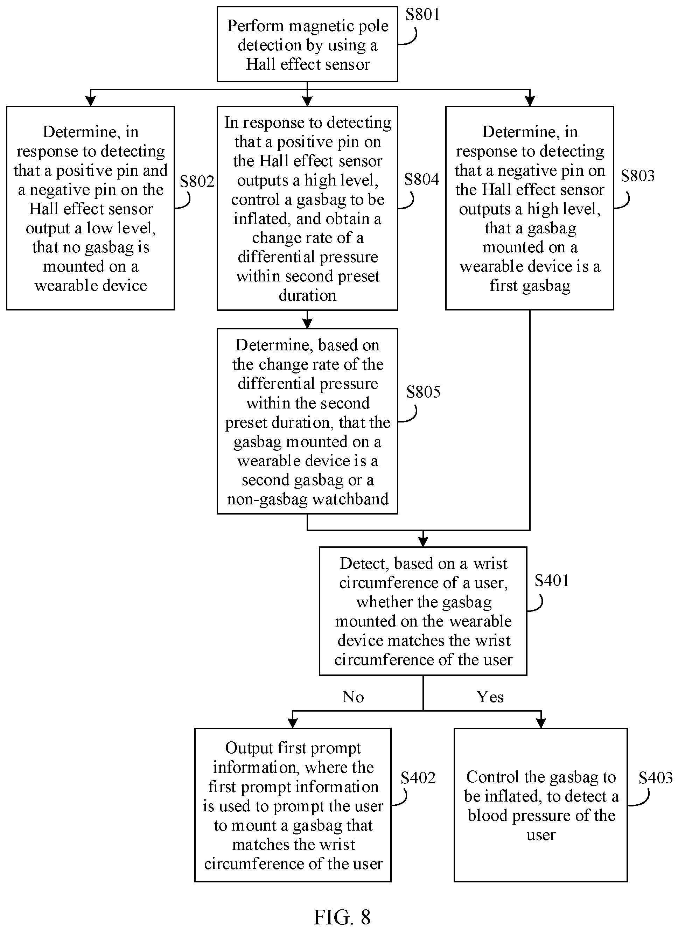

It should be understood that before detecting whether the gasbag mounted on the wearable device matches the wrist circumference, the wearable device may first detect whether the gasbag is mounted on the wearable device. When the gasbag is mounted on the wearable device, the wearable device may detect whether the gasbag mounted on the wearable device matches the wrist circumference. The wearable device detects whether the gasbag is mounted on the wearable device, which may refer to the related descriptions of FIG. 2, FIG. 3, and FIG. 7, and related descriptions of FIG. 8.

S402: The wearable device outputs first prompt information, where the first prompt information is used to prompt the user to mount the gasbag that matches the wrist circumference of the user.

As shown in a in FIG. 5, after the user inputs the wrist circumference in the input box 511, if the wearable device detects that the gasbag mounted on the wearable device does not match the wrist circumference, the wearable device may output the first prompt information. The first prompt information is used to prompt the user to mount the gasbag that matches the wrist circumference of the user, and the first prompt information may include an identifier of the gasbag that matches the wrist circumference of the user. The identifier of the gasbag that matches the wrist circumference of the user may be information used to distinguish the gasbag, such as a type of the gasbag or a volume of the gasbag.

In an embodiment, a manner in which the wearable device outputs the first prompt information may be: The wearable device outputs the first prompt information in a voice broadcast manner, or displays the first prompt information on the interface displayed on the wearable device. A manner in which the wearable device outputs the first prompt information is not limited in embodiments of this application. As shown in b in FIG. 5, the manner in which the wearable device displays the first prompt information on the interface is used as an example for description. For example, the wearable device may display, on the interface 51 displayed on the wearable device, a prompt box 513 including the first prompt information, where the prompt box 513 displays "The current gasbag does not match the wrist circumference. Please replace the current gasbag with the second gasbag (the large-sized gasbag) that matches the wrist circumference". Similarly, if the wearable device detects that the gasbag mounted on the wearable device does not match the wrist circumference, as shown in b in FIG. 6, the wearable device may display, on the interface 51, the prompt box 513 including the first prompt information.

In this embodiment, the user may remove the gasbag that does not match the wrist circumference of the user, and mount the wearable device that matches the wrist circumference of the user. After mounting the wearable device that matches the wrist circumference of the user, the user may operate the measurement control 512 to continue to trigger the wearable device to start to measure the blood pressure. In this way, the wearable device may perform S401B, to determine that the gasbag mounted on the wearable device matches the wrist circumference, and then perform S403.

S403: The wearable device controls the gasbag to be inflated, to detect the blood pressure of the user.

In an embodiment, the wearable device may control an air pump to start working, so that the air pump may inflate the gasbag through an air nozzle. A process in which the wearable device inflates the gasbag and a process of detecting the blood pressure of the user are not described in this embodiment of this application. For details, refer to related descriptions in the conventional technology.

FIG. 5 is used as an example. After the user mounts the wearable device that matches the wrist circumference of the user, the wearable device may perform S403. After measuring the blood pressure of the user, the wearable device may display a blood pressure measurement result on an interface of the wearable device, which is not shown in FIG. 5.

In this embodiment of this application, when the user inputs the wrist circumference to the wearable device or triggers the wearable device to measure the blood pressure, the wearable device may detect, based on the wrist circumference of the user, whether the gasbag mounted on the wearable device matches the wrist circumference of the user. When the gasbag mounted on the wearable device does not match the wrist circumference, the user may be prompted to mount the gasbag that matches the wrist circumference of the user, to improve accuracy of blood pressure measurement.

In the embodiment, the wearable device detects the type of the gasbag by detecting the level output by the Hall effect sensor disposed in the wearable device. The type of the gasbag is detected by the Hall effect sensor based on the level output by the positive pin 211 or the negative pin 212 on the Hall effect sensor 21, and the type of the gasbag that can be identified by the Hall effect sensor is limited, for example, the second gasbag due to which the positive pin 211 outputs the high level, and the first gasbag due to which the negative pin 212 outputs the high level. Consequently, an application range is small, and the type of the gasbag supported by the wearable device is limited.

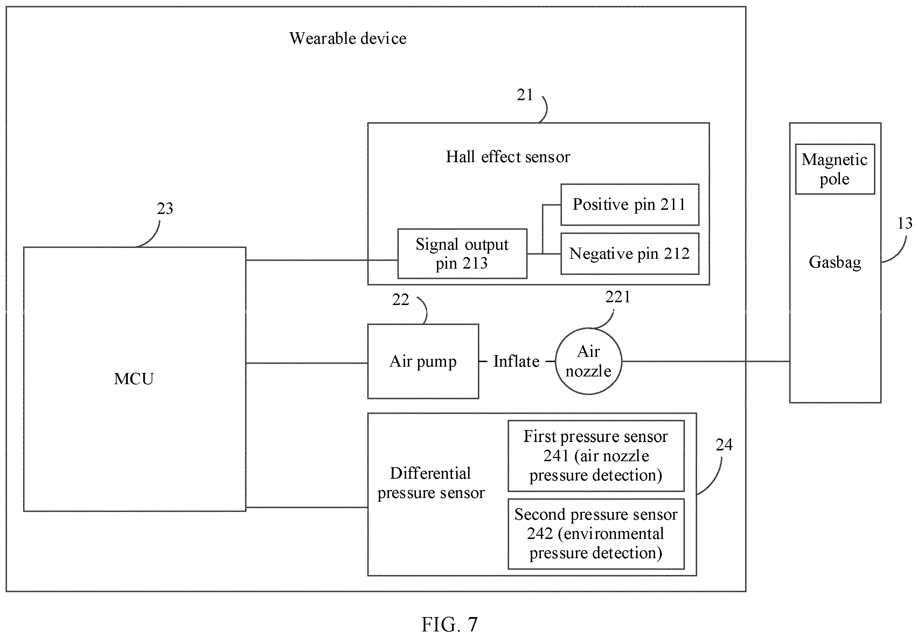

As shown in FIG. 2 and FIG. 7, in an embodiment, compared with FIG. 2, a differential pressure sensor 24 is further disposed on the wearable device, and the MCU 23 may be connected to the differential pressure sensor 24. In an embodiment, the MCU 23 may be connected to the differential pressure sensor 24 through an inter-integrated circuit (inter-integrated circuit, I2C) bus. The differential pressure sensor 24 is configured to detect a differential pressure at the air nozzle 221.

In this embodiment, in response to receiving an instruction indicating that the user inputs a wrist circumference or an instruction that is input by the user and for measuring blood pressure, the MCU 23 may control the air pump 22 to blow air into the gasbag at a preset rate through the air nozzle 221, to detect the type of the gasbag based on a change rate of the differential pressure of pressures collected by a first pressure sensor 241 and a second pressure sensor 242. For details, refer to the following descriptions.

The differential pressure sensor 24 may include the first pressure sensor 241 and the second pressure sensor 242. In an embodiment, the first pressure sensor 241 is disposed at the air nozzle 221, and the second pressure sensor 242 communicates with an external environment. Specifically, the MCU 23 may be separately connected to the first pressure sensor 241 and the second pressure sensor 242. The first pressure sensor 241 is configured to detect a first pressure at the air nozzle 221, and the second pressure sensor 242 is configured to detect an environmental pressure, where the environmental pressure is a second pressure.

In a process in which the air pump 22 blows air to the gasbag through the air nozzle 221 at the preset rate, the first pressure sensor 241 is configured to detect once the first pressure at a first location at an interval of first preset duration. For example, the first preset duration is 5 ms. The second pressure sensor 242 is configured to detect once the second pressure at a second location at the interval of first preset duration. It should be understood that the first location is a location of the first pressure sensor 241, and the second location is a location of the second pressure sensor 242.

In other words, within second preset duration, the first pressure sensor 241 may collect the first pressure N1 times, and the second pressure sensor 242 may collect the second pressure N1 times. N1 is an integer greater than or equal to 1.

The MCU 23 may obtain an average value of first pressures collected by the first pressure sensor 241 within the second preset duration, namely, a first pressure average value, or may obtain an average value of second pressures collected by the second pressure sensor 242 within the second preset duration, namely, a second pressure average value. For example, the second preset duration may be 1s.

The MCU 23 obtains a change rate of the differential pressure within the second preset duration based on the first pressure average value and the second pressure average value. For example, the MCU 23 obtains the change rate of the differential pressure within the second preset duration, which may refer to Formula 1.

$$V_p = \frac{P_1 - P_2}{T} \quad \text{Formula 1}$$

Herein, $V_p$ is the change rate of the differential pressure, $P_1$ is the first pressure average value, $P_2$ is the second pressure average value, and T is the second preset duration.

The MCU 23 may detect the type of the gasbag based on the change rate of the differential pressure within the second preset duration. For details, refer to related descriptions in the following embodiment.

Based on the structure of the wearable device in FIG. 7, in this embodiment, because the MCU 23 is included in the wearable device, the following uses an example in which the wearable device is an execution body for description.

In an embodiment, the wearable device stores a mapping relationship between a change rate of a differential pressure and a type of a gasbag. It may be understood that a smaller volume of the gasbag indicates a larger change rate of the differential pressure within the second preset duration. When the non-gasbag watchband is disposed on the wearable device, the non-gasbag watchband blocks the air nozzle 221. In this case, in the process in which the air pump 22 blows air to the gasbag through the air nozzle 221 at the preset rate, a large differential pressure is generated in short time. Therefore, the change rate of the differential pressure is large in the short time. When no non-gasbag watchband is mounted on the wearable device, or no gasbag is mounted on the wearable device, the air nozzle 221 communicates with air. Therefore, the change rate of the differential pressure within the second preset duration is 0.

In other words, if the change rate of the differential pressure within the second preset duration is 0, or the change rate of the differential pressure within the second preset duration is greater than or equal to a first change rate threshold, the wearable device determines that no gasbag is mounted on the wearable device. If the change rate of the differential pressure within the second preset duration is less than the first change rate threshold, the wearable device determines that the gasbag is mounted on the wearable device. The first change rate threshold may be a predefined value.

In an embodiment, the mapping relationship between the change rate of the differential pressure and the type of the gasbag may be shown in Table 2.

**Table 2**

| Change rate of the differential pressure | Type of the gasbag |
|---|---|
| a1 to b1 | First gasbag |
| b1 to c1 | Second gasbag |
| c1 to d1 | Third gasbag |
| ≥e | Non-gasbag watchband |
| 0 | No gasbag or non-gasbag watchband |

In this embodiment of this application, in response to information that the change rate of the differential pressure within the second preset duration is less than the first change rate threshold, the wearable device may detect the type of the gasbag based on the change rate of the differential pressure within the second preset duration and the mapping relationship between the change rate of the differential pressure and the type of the gasbag. After obtaining the type of the gasbag, the wearable device may detect whether the gasbag matches a wrist circumference of the user. For details, refer to the related descriptions in the foregoing embodiments.

In this embodiment of this application, the differential pressure sensor is disposed on the wearable device. The wearable device may blow air by using the air pump, detect the change rate of the differential pressure within the second preset duration by using the differential pressure sensor, and detect the type of the gasbag based on the change rate of the differential pressure and the mapping relationship between the change rate of the differential pressure and the type of the gasbag. After obtaining the type of the gasbag, the wearable device may detect whether the gasbag matches the wrist circumference of the user. In this embodiment, the type of the gasbag may be detected based on the change rate of the differential pressure. This is applicable to a plurality of types of gasbags, and is not limited to two gasbags (in Table 2, three types of gasbags are used as an example for description). An application range is wide. When the gasbag mounted on the wearable device does not match the wrist circumference, the user may still be prompted to mount a gasbag that matches the wrist circumference of the user, to improve accuracy of blood pressure measurement.

Based on the structure of the wearable device shown in FIG. 7, in an embodiment, the wearable device may combine a manner of detecting the type of the gasbag by using the Hall effect sensor with a manner of detecting the type of the gasbag based on the change rate of the differential pressure, to detect whether the gasbag matches the wrist circumference of the user.

In this embodiment, a same magnetic pole may be disposed on gasbags mounted on the wearable device, or at least one gasbag is disposed with a same magnetic pole as the non-gasbag watchband. In this embodiment, to accurately detect whether the gasbag is mounted on the wearable device and the type of the gasbag mounted on the wearable device, the manner of detecting the type of the gasbag by using the Hall effect sensor needs to be combined with the manner of detecting the type of the gasbag based on the change rate of the differential pressure.

In other words, the wearable device may detect, based on the level output through the signal output pin and the change rate of the differential pressure within the second preset duration, whether the gasbag is mounted on the wearable device, and the wearable device may detect, based on the pin (the positive pin or the negative pin) that outputs, through the signal output pin, the level greater than the second threshold and the change rate of the differential pressure within the second preset duration, the identifier (type) of the gasbag mounted on the wearable device.

In an embodiment, the negative pole is disposed on the first gasbag, the positive pole is disposed on the second gasbag, and the positive pole is disposed on the non-gasbag watchband.

As shown in FIG. 8, in this embodiment, the gasbag detection method for a wearable device provided in embodiments of this application may include the following steps.

S801: The wearable device performs magnetic pole detection by using the Hall effect sensor.

That the wearable device performs magnetic pole detection by using the Hall effect sensor may be understood as follows: The wearable device detects whether the positive pin or the negative pin on the Hall effect sensor outputs the high level, so that when detecting that the pin outputs the high level, the wearable device can obtain a magnetic pole detected by the pin that outputs the high level.

In this embodiment, the negative pole is disposed on the first gasbag, the positive pole is disposed on the second gasbag, and the positive pole is disposed on the non-gasbag watchband. When no non-gasbag watchband is mounted on the wearable device, or only the gasbag watchband is mounted on the wearable device, but no gasbag is mounted, the positive pin and the negative pin on the Hall effect sensor do not detect approaching of the magnetic pole. Therefore, the positive pin and the negative pin may output a low level through the signal output pin.

When the user mounts the first gasbag on the gasbag watchband, and mounts the gasbag watchband on the wearable device, the negative pole of the first gasbag is in contact with the negative pin on the Hall effect sensor, and the negative pin detects the negative pole. Therefore, a high level may be output through the signal output pin. When the user mounts the second gasbag on the gasbag watchband and mounts the gasbag watchband on the watch head, the positive pole of the second gasbag is in contact with the positive pin on the Hall effect sensor, and the positive pin detects the positive pole. Therefore, a high level may be output through the signal output pin. Because the positive pole is disposed on the non-gasbag watchband, when the user mounts the gasbag watchband on the watch head, the positive pole of the non-gasbag watchband is in contact with the positive pin on the Hall effect sensor, and the positive pin detects the positive pole. Therefore, a high level may be output through the signal output pin.

In an embodiment, S801 may be replaced with S801A or S801B.

S801A: The wearable device performs, in response to detecting the instruction indicating that the user inputs the wrist circumference, magnetic pole detection by using the Hall effect sensor.

S801B: The wearable device performs, in response to detecting that the user inputs the instruction for measuring the blood pressure, magnetic pole detection by using the Hall effect sensor.

S802: The wearable device determines, in response to detecting that the positive pin and the negative pin on the Hall effect sensor output a low level, that no gasbag is mounted on the wearable device.

As described in S801, in response to detecting that the positive pin and the negative pin on the Hall effect sensor output a low level, the wearable device may determine that the non-gasbag watchband is mounted on the wearable device, or that only the gasbag watchband is mounted on the wearable device, and no gasbag is mounted on the wearable device, that is, no gasbag is mounted on the wearable device.

In an embodiment, when determining that no gasbag is mounted on the wearable device, the wearable device may output second prompt information. The second prompt information is used to prompt the user to mount the gasbag. After the user mounts the gasbag, the wearable device may return to perform S801.

S803: The wearable device determines, in response to detecting that the negative pin on the Hall effect sensor outputs a high level, that the gasbag mounted on the wearable device is the first gasbag.

As described in S801, the wearable device determines, in response to detecting that the negative pin on the Hall effect sensor outputs a high level, that the gasbag mounted on the wearable device is the first gasbag.

S804: In response to detecting that the positive pin on the Hall effect sensor outputs a high level, the wearable device controls the gasbag to be inflated, and obtains the change rate of the differential pressure within the second preset duration.

Because the positive pole is disposed on both the second gasbag and the non-gasbag watchband, when the user mounts the second gasbag or the non-gasbag watchband on the wearable device, the positive pin on the Hall effect sensor outputs a high level. In this way, when detecting that the positive pin on the Hall effect sensor outputs a high level, the wearable device may determine that the second gasbag or the non-gasbag watchband is mounted on the wearable device, but cannot determine whether the second gasbag or the non-gasbag watchband is specifically mounted on the wearable device. Therefore, the wearable device needs to determine, with reference to the detection manner based on the change rate of the differential pressure, whether the second gasbag or the non-gasbag watchband is specifically mounted on the wearable device.

Therefore, in this embodiment, in response to detecting that the positive pin on the Hall effect sensor outputs a high level, the wearable device controls the gasbag to be inflated, and obtains the change rate of the differential pressure within the second preset duration. The wearable device controls the gasbag to be inflated, and obtains the change rate of the differential pressure within the second preset duration, which may refer to the related descriptions in the foregoing embodiments.

S805: The wearable device determines, based on the change rate of the differential pressure within the second preset duration, that the gasbag mounted on the wearable device is the second gasbag or the non-gasbag watchband.

For example, the wearable device may determine, based on the mapping relationship (as shown in Table 2) between the change rate of the differential pressure and the type of the gasbag, that the gasbag mounted on the wearable device is the second gasbag or the non-gasbag watchband.

One of S802, S803, and S804 and S805 is selected and performed, and S401 to S403 may be performed after S803 and S805. Because the type of the gasbag is detected and obtained in this embodiment of this application, for performing S401, "The wearable device detects, based on a wrist circumference of a user, whether a gasbag mounted on the wearable device matches the wrist circumference" in S401 is performed. For details, refer to the related descriptions in the foregoing embodiments.

In this embodiment of this application, the wearable device may accurately detect, with reference to the magnetic pole detection manner based on the Hall effect sensor and the detection manner based on the change rate of the pressure, the type of the gasbag disposed on the wearable device, to detect whether the gasbag disposed on the wearable device matches the wrist circumference of the user. When the gasbag mounted on the wearable device does not match the wrist circumference, the user may be prompted to mount the gasbag that matches the wrist circumference of the user, to improve accuracy of blood pressure measurement.

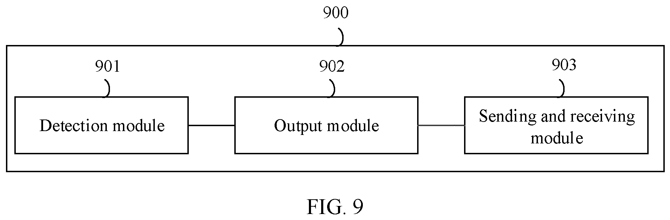

FIG. 9 is a schematic diagram of a structure of a gasbag detection apparatus according to an embodiment of this application. The gasbag detection apparatus in this embodiment may be the wearable device, or may be a chip applied to the wearable device. The gasbag detection apparatus may be configured to perform actions of the wearable device in the foregoing method embodiments. As shown in FIG. 9, the gasbag detection apparatus 900 may include a detection module 901, an output module 902, and a sending and receiving module 903.

The detection module 901 is configured to: when a gasbag is mounted on the wearable device, detect, based on a wrist circumference of a user, whether the gasbag mounted on the wearable device matches the wrist circumference of the user.

The output module 902 is configured to: if the gasbag mounted on the wearable device does not match the wrist circumference of the user, output first prompt information, where the first prompt information indicates to mount a gasbag that matches the wrist circumference of the user.

In a possible implementation, the detection module 901 is further configured to detect whether the gasbag is mounted on the wearable device.

The detection module 901 is specifically configured to: in response to detecting that the gasbag is mounted on the wearable device, detect an identifier of the gasbag mounted on the wearable device, and detect, based on a mapping relationship between a wrist circumference and an identifier of a gasbag, whether the identifier of the gasbag mounted on the wearable device is an identifier of a gasbag mapped to the wrist circumference of the user.

In a possible implementation, the detection module 901 is specifically configured to: if the identifier of the gasbag mounted on the wearable device is not the identifier of the gasbag mapped to the wrist circumference of the user, determine that the gasbag mounted on the wearable device does not match the wrist circumference of the user; or if the identifier of the gasbag mounted on the wearable device is the identifier of the gasbag mapped to the wrist circumference of the user, determine that the gasbag mounted on the wearable device matches the wrist circumference of the user.

The wearable device includes a Hall effect sensor, and the Hall effect sensor includes a positive pin, a negative pin, and a signal output pin.

In response to sensing a positive magnetic pole, the positive pin outputs, through the signal output pin, a level greater than a second threshold; in response to sensing a negative magnetic pole, the negative pin outputs, through the signal output pin, the level greater than the second threshold; when the positive pin does not sense the positive magnetic pole, the positive pin outputs, through the signal output pin, a level less than a first threshold, where the second threshold is greater than the first threshold; and when the negative pin does not sense the negative magnetic pole, the negative pin outputs, through the signal output pin, the level less than the first threshold.

In a possible implementation, the wearable device includes at least two gasbags, magnetic poles are disposed on the gasbags of the at least two gasbags, and a different magnetic pole is disposed on each gasbag.

The detection module 901 is specifically configured to: detect, based on the level output through the signal output pin, whether the gasbag is mounted on the wearable device; and detect, based on the pin that outputs, through the signal output pin, the level greater than the second threshold, the identifier of the gasbag mounted on the wearable device.

In a possible implementation, the at least two gasbags include a first gasbag and a second gasbag, the negative magnetic pole is disposed on the first gasbag, and the positive magnetic pole is disposed on the second gasbag.

The detection module 901 is specifically configured to: if the level output through the signal output pin is less than the first threshold, determine that no gasbag is mounted on the wearable device; or if the level output through the signal output pin is greater than the second threshold, determine that the gasbag is mounted on the wearable device.

In a possible implementation, if the positive pin outputs, through the signal output pin, the level greater than the second threshold, it is determined that the gasbag mounted on the wearable device is the second gasbag; or if the negative pin outputs, through the signal output pin, the level greater than the second threshold, the detection module 901 is specifically configured to determine that the gasbag mounted on the wearable device is the first gasbag.

In a possible implementation, the wearable device includes a differential pressure sensor and a gasbag watchband, the differential pressure sensor is configured to detect a differential pressure at an air nozzle of an air pump in the wearable device, the gasbag is mounted on the gasbag watchband, and when the gasbag watchband on which the gasbag is mounted is mounted on the wearable device, an air intake on the gasbag communicates with the air nozzle.

The detection module 901 is specifically configured to: control the air pump to blow air at a preset rate; obtain a change rate of the differential pressure within second preset duration based on the differential pressure at the air nozzle collected by the differential pressure sensor; detect, based on the change rate of the differential pressure within the second preset duration, whether the gasbag is mounted on the wearable device; and detect, based on the change rate of the differential pressure within the second preset duration, the identifier of the gasbag mounted on the wearable device.

In a possible implementation, the wearable device further includes a non-gasbag watchband, and when the non-gasbag watchband is mounted on the wearable device, the non-gasbag watchband blocks the air nozzle of the air pump.

If the change rate of the differential pressure within the second preset duration is 0, or the change rate of the differential pressure within the second preset duration is greater than or equal to a first change rate threshold, the detection module 901 is specifically configured to determine that no gasbag is mounted on the wearable device; or

if the change rate of the differential pressure within the second preset duration is less than the first change rate threshold, the detection module 901 is specifically configured to determine that the gasbag is mounted on the wearable device.

In a possible implementation, the detection module 901 is specifically configured to: in response to information that the change rate of the differential pressure within the second preset duration is less than the first change rate threshold, detect, based on the change rate of the differential pressure within the second preset duration and a mapping relationship between a change rate of a differential pressure and an identifier of a gasbag, the identifier of the gasbag mounted on the wearable device.

In a possible implementation, the wearable device includes: a differential pressure sensor, a gasbag watchband, and at least two gasbags, the differential pressure sensor is configured to detect a differential pressure at an air nozzle of an air pump in the wearable device, the gasbag is mounted on the gasbag watchband, when the gasbag watchband on which the gasbag is mounted is mounted on the wearable device, an air intake on the gasbag communicates with the air nozzle, and magnetic poles disposed on at least two of the following are the same: a first gasbag in the at least two gasbags, a second gasbag in the at least two gasbags, and a non-gasbag watchband.

The detection module 901 is specifically configured to: control the air pump to blow air at a preset rate; obtain a change rate of the differential pressure within second preset duration based on the differential pressure at the air nozzle collected by the differential pressure sensor; detect, based on the level output through the signal output pin and the change rate of the differential pressure within the second preset duration, whether the gasbag is mounted on the wearable device;

and detect, based on the pin that outputs, through the signal output pin, the level greater than the second threshold and the change rate of the differential pressure within the second preset duration, the identifier of the gasbag mounted on the wearable device.

In a possible implementation, the negative magnetic pole is disposed on the first gasbag, the positive magnetic pole is disposed on the second gasbag, and the positive magnetic pole is disposed on the non-gasbag watchband.

The detection module 901 is specifically configured to: if the level output through the signal output pin is less than the first threshold, determine that no gasbag is mounted on the wearable device; if the level output through the signal output pin is greater than the second threshold, and the negative pin outputs, through the signal output pin, the level greater than the second threshold, determine that the gasbag mounted on the wearable device is the first gasbag; or if the level output through the signal output pin is greater than the second threshold, and the positive pin outputs, through the signal output pin, the level greater than the second threshold, detect, based on the change rate of the differential pressure within the second preset duration, that the second gasbag or the non-gasbag watchband is mounted on the wearable device.

In a possible implementation, when the non-gasbag watchband is mounted on the wearable device, the non-gasbag watchband blocks the air nozzle of the air pump.

The detection module 901 is specifically configured to: if the change rate of the differential pressure within the second preset duration is greater than or equal to a first change rate threshold, determine that the non-gasbag watchband is mounted on the wearable device; or if the change rate of the differential pressure within the second preset duration is less than the first change rate threshold, determine that the gasbag mounted on the wearable device is the second gasbag.

In a possible implementation, the differential pressure sensor includes a first pressure sensor and a second pressure sensor, the first pressure sensor is configured to detect a first pressure at the air nozzle, the second pressure sensor is configured to detect a second pressure, the second pressure is an environmental pressure, and the differential pressure at the air nozzle is a difference between the first pressure and the second pressure.

The detection module 901 is specifically configured to: obtain once the first pressure collected by the first pressure sensor at an interval of first preset duration; obtain once the second pressure collected by the second pressure sensor at the interval of first preset duration; obtain a first pressure average value based on first pressures collected within the second preset duration; obtain a second pressure average value based on second pressures collected within the second preset duration; and obtain the change rate of the differential pressure within the second preset duration based on the first pressure average value and the second pressure average value.

In a possible implementation, the sending and receiving module 903 is configured to receive an instruction indicating that the user inputs the wrist circumference, or receive an instruction that is input by the user and that is for measuring a blood pressure.

In a possible implementation, the first prompt information includes the identifier of the gasbag that matches the wrist circumference of the user.

The gasbag detection apparatus provided in this embodiment of this application may perform actions of the wearable device in the foregoing method embodiments. Implementation principles and technical effects thereof are similar. Details are not described herein again.

The disclosure further relates to a wearable device, including a Hall effect sensor. The Hall effect sensor includes a positive pin, a negative pin, and a signal output pin.

In response to sensing a positive magnetic pole, the positive pin outputs, through the signal output pin, a level greater than a second threshold; in response to sensing a negative magnetic pole, the negative pin outputs, through the signal output pin, the level greater than the second threshold; when the positive pin does not sense the positive magnetic pole, the positive pin outputs, through the signal output pin, a level less than a first threshold, where the second threshold is greater than the first threshold; and when the negative pin does not sense the negative magnetic pole, the negative pin outputs, through the signal output pin, the level less than the first threshold.

In a possible implementation, the wearable device includes at least two gasbags and a microprocessor unit MCU, and the MCU is connected to the Hall effect sensor; and the MCU is configured to detect the level output through the signal output pin.

In a possible implementation, magnetic poles are disposed on the gasbags of the at least two gasbags, and a different magnetic pole is disposed on each gasbag.

The MCU is further configured to: detect, based on the level output through the signal output pin, whether the gasbag is mounted on the wearable device; and detect, based on the pin that outputs, through the signal output pin, the level greater than the second threshold, an identifier of the gasbag mounted on the wearable device.

In a possible implementation, the wearable device further includes a differential pressure sensor and a gasbag watchband, the differential pressure sensor is configured to detect a differential pressure at an air nozzle of an air pump in the wearable device, the gasbag is mounted on the gasbag watchband, and when the gasbag watchband on which the gasbag is mounted is mounted on the wearable device, an air intake on the gasbag communicates with the air nozzle.

The MCU is further configured to: control the air pump to blow air at a preset rate; obtain a change rate of the differential pressure within second preset duration based on the differential pressure at the air nozzle collected by the differential pressure sensor; detect, based on the change rate of the differential pressure within the second preset duration, whether the gasbag is mounted on the wearable device; and detect, based on the change rate of the differential pressure within the second preset duration, the identifier of the gasbag mounted on the wearable device.

In a possible implementation, the wearable device further includes: a differential pressure sensor, a gasbag watchband, and the at least two gasbags, the differential pressure sensor is configured to detect a differential pressure at an air nozzle of an air pump in the wearable device, the gasbag is mounted on the gasbag watchband, when the gasbag watchband on which the gasbag is mounted is mounted on the wearable device, an air intake on the gasbag communicates with the air nozzle, and magnetic poles disposed on at least two of the following are the same: a first gasbag in the at least two gasbags, a second gasbag in the at least two gasbags, and a non-gasbag watchband.

The MCU is further configured to: control the air pump to blow air at a preset rate; obtain a change rate of the differential pressure within second preset duration based on the differential pressure at the air nozzle collected by the differential pressure sensor; detect, based on the level output through the signal output pin and the change rate of the differential pressure within the second preset duration, whether the gasbag is mounted on the wearable device; and detect, based on the pin that outputs, through the signal output pin, the level greater than the second threshold and the change rate of the differential pressure within the second preset duration, an identifier of the gasbag mounted on the wearable device.

In a possible implementation, the differential pressure sensor includes a first pressure sensor and a second pressure sensor, the first pressure sensor is disposed at the air nozzle, the first pressure sensor is configured to detect a first pressure at the air nozzle, the second pressure sensor is configured to detect a second pressure, the second pressure is an environmental pressure, and the differential pressure at the air nozzle is a difference between the first pressure and the second pressure.

In a possible implementation, the positive pin, the negative pin, and the air nozzle are disposed on an outer surface of the wearable device.

It should be understood that the wearable device provided in this embodiment of this application may perform actions of the wearable device in the foregoing method embodiments. Implementation principles and technical effects thereof are similar. Details are not described herein again.

In an embodiment, an embodiment of this application further provides a wearable device. The wearable device may include a processor and a memory. The memory may include a high speed random-access memory (random-access memory, RAM), and may further include a non-volatile memory (non-volatile memory, NVM), for example, at least one disk memory. The memory may store various instructions, to complete various processing functions and implement the method steps of this application. It should be understood that, in an embodiment, the processor may not be limited to an MCU or a CPU. As shown in FIG. 2, in an embodiment, the wearable device may further include a Hall effect sensor and an air pump. As shown in FIG. 7, in an embodiment, the wearable device may further include a Hall effect sensor, an air pump, and a differential pressure sensor.

Optionally, the wearable device in this application may further include a power supply, a communication bus, and a communication port. The communications port is configured to implement connection and communication between the wearable device and another peripheral. In this embodiment of this application, the memory is configured to store computer-executable program code. The program code includes instructions. When the processor executes the instructions, the instructions enable the processor of the wearable device to perform the actions in the foregoing method embodiments. Implementation principles and technical effects thereof are similar. Details are not described herein again.

It should be noted that the modules or components in the foregoing embodiments may be one or more integrated circuits configured to implement the foregoing method, for example, one or more application-specific integrated circuits (application-specific integrated circuit, ASIC), one or more microprocessors (digital signal processor, DSP), or one or more field programmable gate arrays (field programmable gate array, FPGA). For another example, when one of the foregoing modules is implemented in a form of scheduling program code by a processing element, the processing element may be a general-purpose processor, for example, a central processing unit (central processing unit, CPU) or another processor that can invoke the program code, for example, a controller. For another example, the modules may be integrated together, and implemented in a form of a system-on-a-chip (system-on-a-chip, SOC).

All or some of the foregoing embodiments may be implemented by using software, hardware, firmware, or any combination thereof. When software is used to implement embodiments, all or a part of embodiments may be implemented in a form of a computer program product. The computer program product includes one or more computer instructions. When the computer program instructions are loaded and executed on a computer, all or some of the procedures or functions according to embodiments of this application are generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk drive, or a magnetic tape), an optical medium (for example, a DVD), a semiconductor medium (for example, a solid state disk (Solid State Disk, SSD)), or the like.

The term "a plurality of" in this specification means two or more than two. The term "and/or" in this specification describes only an association relationship for describing associated objects and represents that three relationships may exist. For example, A and/or B may represent the following three cases: Only A exists, both A and B exist, and only B exists. In addition, the character "/" in this specification usually indicates an "or" relationship between the associated objects. In the formula, the character "/" indicates a "division" relationship between the associated objects. In addition, it should be understood that in description of this application, terms such as "first" and "second" are merely used for distinguishing and description, but should not be understood as indicating or implying relative importance, or should not be understood as indicating or implying a sequence.

It may be understood that various numbers in embodiments of this application are merely used for differentiation for ease of description, and are not used to limit the scope of embodiments of this application.

It should be understood that sequence numbers of the foregoing processes do not mean execution sequences in embodiments of this application. The execution sequences of the processes should be determined based on functions and internal logic of the processes, and should not be construed as any limitation on the implementation processes of embodiments of this application. The invention is defined by the appended claims.

**Claims**

1.  A computer-implemented gasbag (13) detection method for a wearable device, comprising:

    detecting whether the gasbag (13) is mounted on the wearable device;
    when a gasbag (13) is mounted on the wearable device, detecting (S401), based on a wrist circumference of a user, whether the gasbag (13) mounted on the wearable device matches the wrist circumference of the user, comprising:

    in response to detecting that the gasbag (13) is mounted on the wearable device, detecting an identifier of the gasbag (13) mounted on the wearable device; and
    detecting, based on a mapping relationship between a wrist circumference and an identifier of a gasbag (13), whether the identifier of the gasbag (13) mounted on the wearable device is an identifier of a gasbag (13) mapped to the wrist circumference of the user; and

    if the gasbag (13) mounted on the wearable device does not match the wrist circumference of the user, outputting (S402) first prompt information, wherein the first prompt information indicates to mount a gasbag (13) that matches the wrist circumference of the user;
    wherein the wearable device comprises a Hall effect sensor (21), an air pump (22), and a micro controller unit, MCU, (23), the MCU (23) being separately connected to the Hall effect sensor (21) and the air pump (22);
    wherein the Hall effect sensor (21) comprises a positive pin (211), a negative pin (212), and a signal output pin (213);
    wherein the air pump (22) is disposed in the wearable device, and an air nozzle (221) connected to the air pump (22) is disposed on the outer surface of the wearable device;
    wherein an air intake is disposed on the gasbag (13), and when the gasbag (13) is mounted on the wearable device, the air nozzle (221) is configured to communicate with the air intake of the gasbag (13).

2.  The method according to claim 1, wherein the detecting, based on a mapping relationship between a wrist circumference and an identifier of a gasbag (13), whether the identifier of the gasbag (13) mounted on the wearable device is an identifier of a gasbag (13) mapped to the wrist circumference of the user comprises:

    if the identifier of the gasbag (13) mounted on the wearable device is not the identifier of the gasbag (13) mapped to the wrist circumference of the user, determining that the gasbag (13) mounted on the wearable device does not match the wrist circumference of the user; or
    if the identifier of the gasbag (13) mounted on the wearable device is the identifier of the gasbag (13) mapped to the wrist circumference of the user, determining that the gasbag (13) mounted on the wearable device matches the wrist circumference of the user.

3.  The method according to claim 1 or 2, wherein :

in response to sensing a positive magnetic pole, the positive pin (211) outputs, through the signal output pin (213), a level greater than a second threshold;

in response to sensing a negative magnetic pole, the negative pin (212) outputs, through the signal output pin (213), the level greater than the second threshold;

when the positive pin (211) does not sense the positive magnetic pole, the positive pin (211) outputs, through the signal output pin (213), a level less than a first threshold, wherein the second threshold is greater than the first threshold; and

when the negative pin (212) does not sense the negative magnetic pole, the negative pin (212) outputs, through the signal output pin (213), the level less than the first threshold.

4. The method according to claim 3, wherein the wearable device comprises at least two gasbags, magnetic poles are disposed on the gasbags of the at least two gasbags, and a different magnetic pole is disposed on each gasbag (13); the detecting whether the gasbag (13) is mounted on the wearable device comprises:

   detecting, based on the level output through the signal output pin (213), whether the gasbag (13) is mounted on the wearable device; and
   the detecting an identifier of the gasbag (13) mounted on the wearable device comprises:
   detecting, based on the pin that outputs, through the signal output pin (213), the level greater than the second threshold, the identifier of the gasbag (13) mounted on the wearable device.

5. The method according to claim 4, wherein the at least two gasbags comprise a first gasbag (13) and a second gasbag (13), the negative magnetic pole is disposed on the first gasbag (13), the positive magnetic pole is disposed on the second gasbag (13), and the detecting, based on the level output through the signal output pin (213), whether the gasbag (13) is mounted on the wearable device comprises:

   if the level output through the signal output pin (213) is less than the first threshold, determining that no gasbag (13) is mounted on the wearable device; or
   if the level output through the signal output pin (213) is greater than the second threshold, determining that the gasbag (13) is mounted on the wearable device.

6. The method according to claim 5, wherein the detecting, based on the pin that outputs, through the signal output pin (213), the level greater than the second threshold, the identifier of the gasbag (13) mounted on the wearable device comprises:

   if the positive pin (211) outputs, through the signal output pin (213), the level greater than the second threshold, determining that the gasbag (13) mounted on the wearable device is the second gasbag (13); or
   if the negative pin (212) outputs, through the signal output pin (213), the level greater than the second threshold, determining that the gasbag (13) mounted on the wearable device is the first gasbag (13).

7. The method according to any one of claims 1 to 3, wherein the wearable device comprises a differential pressure sensor (24) and a gasbag watchband (12), the differential pressure sensor (24) is configured to detect a differential pressure at the air nozzle (221) of the air pump (22) in the wearable device, the gasbag (13) is mounted on the gasbag watchband (12), and when the gasbag watchband (12) on which the gasbag (13) is mounted is mounted on the wearable device, an air intake on the gasbag (13) communicates with the air nozzle (221); the detecting whether the gasbag (13) is mounted on the wearable device comprises:

   controlling the air pump (22) to blow air at a preset rate;
   obtaining a change rate of the differential pressure within second preset duration based on the differential pressure at the air nozzle (221) collected by the differential pressure sensor (24); and
   detecting, based on the change rate of the differential pressure within the second preset duration, whether the gasbag (13) is mounted on the wearable device; and
   the detecting an identifier of the gasbag (13) mounted on the wearable device comprises:
   detecting, based on the change rate of the differential pressure within the second preset duration, the identifier of the gasbag (13) mounted on the wearable device.

8. The method according to claim 7, wherein the wearable device further comprises a non-gasbag watchband (12), and when the non-gasbag watchband (12) is mounted on the wearable device, the non-gasbag watchband (12) blocks the air nozzle (221) of the air pump (22); and

the detecting, based on the change rate of the differential pressure within the second preset duration, whether the gasbag (13) is mounted on the wearable device comprises:

if the change rate of the differential pressure within the second preset duration is 0, or the change rate of the differential pressure within the second preset duration is greater than or equal to a first change rate threshold, determining that no gasbag (13) is mounted on the wearable device; or
if the change rate of the differential pressure within the second preset duration is less than the first change rate threshold, determining that the gasbag (13) is mounted on the wearable device.

9. The method according to claim 8, wherein the detecting, based on the change rate of the differential pressure within the second preset duration, the identifier of the gasbag (13) mounted on the wearable device comprises:
in response to information that the change rate of the differential pressure within the second preset duration is less than the first change rate threshold, detecting, based on the change rate of the differential pressure within the second preset duration and a mapping relationship between a change rate of a differential pressure and an identifier of a gasbag (13), the identifier of the gasbag (13) mounted on the wearable device.

10. The method according to claim 3, wherein the wearable device comprises: a differential pressure sensor (24), a gasbag watchband (12), and at least two gasbags (13), the differential pressure sensor (24) is configured to detect a differential pressure at an air nozzle (221) of an air pump (22) in the wearable device, the gasbag (13) is mounted on the gasbag watchband (12), when the gasbag watchband (12) on which the gasbag (13) is mounted is mounted on the wearable device, an air intake on the gasbag (13) communicates with the air nozzle (221), and magnetic poles disposed on at least two of the following are the same: a first gasbag (13) in the at least two gasbags, a second gasbag (13) in the at least two gasbags, and a non-gasbag watchband (12);
the detecting whether the gasbag (13) is mounted on the wearable device comprises:

controlling the air pump (22) to blow air at a preset rate;
obtaining a change rate of the differential pressure within second preset duration based on the differential pressure at the air nozzle (221) collected by the differential pressure sensor (24); and
detecting, based on the level output through the signal output pin (213) and the change rate of the differential pressure within the second preset duration, whether the gasbag (13) is mounted on the wearable device; and
the detecting an identifier of the gasbag (13) mounted on the wearable device comprises:
detecting, based on the pin that outputs, through the signal output pin (213), the level greater than the second threshold and the change rate of the differential pressure within the second preset duration, the identifier of the gasbag (13) mounted on the wearable device.

## Patentansprüche

1. Computerimplementiertes Verfahren zum Erkennen eines Gasbeutels (13) für eine tragbare Vorrichtung, das umfasst:

Erkennen, ob der Gasbeutel (13) an der tragbaren Vorrichtung montiert ist;
wenn ein Gasbeutel (13) an der tragbaren Vorrichtung montiert ist, Erkennen (S401), basierend auf einem Handgelenksumfang eines Benutzers, ob der an der tragbaren Vorrichtung montierte Gasbeutel (13) dem Handgelenksumfang des Benutzers entspricht, das umfasst:

als Reaktion auf das Erkennen, dass der Gasbeutel (13) an der tragbaren Vorrichtung montiert ist, Erkennen einer Kennung des an der tragbaren Vorrichtung montierten Gasbeutels (13); und
Erkennen, basierend auf einer Zuordnungsbeziehung zwischen einem Handgelenksumfang und einer Kennung eines Gasbeutels (13), ob die Kennung des an der tragbaren Vorrichtung montierten Gasbeutels (13) eine Kennung eines Gasbeutels (13) ist, die dem Handgelenksumfang des Benutzers zugeordnet ist; und
falls der an der tragbaren Vorrichtung montierte Gasbeutel (13) nicht dem Handgelenksumfang des Benutzers entspricht, Ausgeben (S402) von ersten Aufforderungsinformationen, wobei die ersten Aufforderungsinformationen angeben, einen Gasbeutel (13) zu montieren, der dem Handgelenksumfang des Benutzers entspricht;
wobei die tragbare Vorrichtung einen Hall-Effekt-Sensor (21), eine Luftpumpe (22) und eine Mikrocontrollereinheit, MCU, (23) umfasst, wobei die MCU (23) separat mit dem Hall-Effekt-Sensor (21) und der Luftpumpe (22) verbunden ist;

wobei der Hall-Effekt-Sensor (21) einen positiven Pin (211), einen negativen Pin (212) und einen Signalausgangs-Pin (213) umfasst;

wobei die Luftpumpe (22) in der tragbaren Vorrichtung angeordnet ist und eine mit der Luftpumpe (22) verbundene Luftdüse (221) auf der Außenoberfläche der tragbaren Vorrichtung angeordnet ist;

wobei ein Lufteinlass an dem Gasbeutel (13) angeordnet ist, und wenn der Gasbeutel (13) an der tragbaren Vorrichtung montiert ist, die Luftdüse (221) konfiguriert ist, um mit dem Lufteinlass des Gasbeutels (13) in Verbindung zu stehen.

2. Verfahren nach Anspruch 1, wobei das Erkennen, basierend auf einer Zuordnungsbeziehung zwischen einem Handgelenksumfang und einer Kennung eines Gasbeutels (13), ob die Kennung des an der tragbaren Vorrichtung montierten Gasbeutels (13) eine Kennung eines Gasbeutels (13) ist, die dem Handgelenksumfang des Benutzers zugeordnet ist, umfasst:

falls die Kennung des an der tragbaren Vorrichtung montierten Gasbeutels (13) nicht die Kennung des Gasbeutels (13) ist, die dem Handgelenksumfang des Benutzers zugeordnet ist, Bestimmen, dass der an der tragbaren Vorrichtung montierte Gasbeutel (13) nicht dem Handgelenksumfang des Benutzers entspricht; oder falls die Kennung des an der tragbaren Vorrichtung montierten Gasbeutels (13) die Kennung des Gasbeutels (13) ist, die dem Handgelenksumfang des Benutzers zugeordnet ist, Bestimmen, dass der an der tragbaren Vorrichtung montierte Gasbeutel (13) dem Handgelenksumfang des Benutzers entspricht.

3. Verfahren nach Anspruch 1 oder 2, wobei:

als Reaktion auf ein Erfassen eines positiven Magnetpols, der positive Pin (211) über den Signalausgangs-Pin (213) einen Pegel ausgibt, der größer als ein zweiter Schwellenwert ist;

als Reaktion auf das Erfassen eines negativen Magnetpols, der negative Pin (212) über den Signalausgangs-Pin (213) den Pegel ausgibt, der größer als der zweite Schwellenwert ist;

wenn der positive Pin (211) den positiven Magnetpol nicht erfasst, der positive Pin (211) über den Signalausgangs-Pin (213) einen Pegel ausgibt, der geringer als ein erster Schwellenwert ist, wobei der zweite Schwellenwert größer als der erste Schwellenwert ist; und

wenn der negative Pin (212) den negativen Magnetpol nicht erfasst, der negative Pin (212) über den Signalausgangs-Pin (213) den Pegel ausgibt, der geringer als der erste Schwellenwert ist.

4. Verfahren nach Anspruch 3, wobei die tragbare Vorrichtung mindestens zwei Gasbeutel umfasst, Magnetpole an den Gasbeuteln der mindestens zwei Gasbeutel angeordnet sind und ein unterschiedlicher Magnetpol an jedem Gasbeutel (13) angeordnet ist;

das Erkennen, ob der Gasbeutel (13) an der tragbaren Vorrichtung montiert ist, umfasst:

Erkennen, basierend auf dem Pegel, der über den Signalausgangs-Pin (213) ausgegeben wird, ob der Gasbeutel (13) an der tragbaren Vorrichtung montiert ist; und

das Erkennen einer Kennung des an der tragbaren Vorrichtung montierten Gasbeutels (13) umfasst:

Erkennen, basierend auf dem Pin, der über den Signalausgangs-Pin (213) den Pegel ausgibt, der größer als der zweite Schwellenwert ist, der Kennung des an der tragbaren Vorrichtung montierten Gasbeutels (13).

5. Verfahren nach Anspruch 4, wobei die mindestens zwei Gasbeutel einen ersten Gasbeutel (13) und einen zweiten Gasbeutel (13) umfassen, der negative Magnetpol an dem ersten Gasbeutel (13) angeordnet ist, der positive Magnetpol an dem zweiten Gasbeutel (13) angeordnet ist, und das Erkennen, basierend auf dem Pegel, der über den Signalausgangs-Pin (213) ausgegeben wird, ob der Gasbeutel (13) an der tragbaren Vorrichtung montiert ist, umfasst:

falls der Pegel, der über den Signalausgangs-Pin (213) ausgegeben wird, geringer als der erste Schwellenwert ist, Bestimmen, dass kein Gasbeutel (13) an der tragbaren Vorrichtung montiert ist; oder falls der Pegel, der über den Signalausgangs-Pin (213) ausgegeben wird, größer als der zweite Schwellenwert ist, Bestimmen, dass der Gasbeutel (13) an der tragbaren Vorrichtung montiert ist.

6. Verfahren nach Anspruch 5, wobei das Erkennen, basierend auf dem Pin, der über den Signalausgangs-Pin (213) den Pegel ausgibt, der größer als der zweite Schwellenwert ist, der Kennung des an der tragbaren Vorrichtung montierten Gasbeutels (13) umfasst:

falls der positive Pin (211) über den Signalausgangs-Pin (213) den Pegel ausgibt, der größer als der zweite Schwellenwert ist, Bestimmen, dass der an der tragbaren Vorrichtung montierte Gasbeutel (13) der zweite Gasbeutel (13) ist; oder

falls der negative Pin (212) über den Signalausgangs-Pin (213) den Pegel ausgibt, der geringer als der zweite Schwellenwert ist, Bestimmen, dass der an der tragbaren Vorrichtung montierte Gasbeutel (13) der erste Gasbeutel (13) ist.

7. Verfahren nach einem der Ansprüche 1 bis 3, wobei die tragbare Vorrichtung einen Differenzdrucksensor (24) und ein Gasbeutel-Uhrenarmband (12) umfasst, der Differenzdrucksensor (24) konfiguriert ist, um einen Differenzdruck an der Luftdüse (221) der Luftpumpe (22) in der tragbaren Vorrichtung zu erkennen, der Gasbeutel (13) an dem Gasbeutel-Uhrenarmband (12) montiert ist, und wenn das Gasbeutel-Uhrenarmband (12), an dem der Gasbeutel (13) montiert ist, an der tragbaren Vorrichtung montiert ist, ein Lufteinlass an dem Gasbeutel (13) mit der Luftdüse (221) in Verbindung steht;

das Erkennen, ob der Gasbeutel (13) an der tragbaren Vorrichtung montiert ist, umfasst:

Steuern der Luftpumpe (22, um Luft mit einer voreingestellten Rate zu blasen;
Erhalten einer Änderungsrate des Differenzdrucks innerhalb einer zweiten voreingestellten Dauer basierend auf dem Differenzdruck an der Luftdüse (221), der durch den Differenzdrucksensor (24) erfasst wird; und
Erkennen, basierend auf der Änderungsrate des Differenzdrucks innerhalb der zweiten voreingestellten Dauer, ob der Gasbeutel (13) an der tragbaren Vorrichtung montiert ist; und
das Erkennen einer Kennung des an der tragbaren Vorrichtung montierten Gasbeutels (13) umfasst:
Erkennen, basierend auf der Änderungsrate des Differenzdrucks innerhalb der zweiten voreingestellten Dauer, der Kennung des an der tragbaren Vorrichtung montierten Gasbeutels (13).

8. Verfahren nach Anspruch 7, wobei die tragbare Vorrichtung ferner ein gasbeutelloses Uhrenarmband (12) umfasst, und wenn das gasbeutellose Uhrenarmband (12) an der tragbaren Vorrichtung montiert ist, blockiert das gasbeutellose Uhrenarmband (12) die Luftdüse (221) der Luftpumpe (22); und

das Erkennen, basierend auf der Änderungsrate des Differenzdrucks innerhalb der zweiten voreingestellten Dauer, ob der Gasbeutel (13) an der tragbaren Vorrichtung montiert ist, umfasst:

falls die Änderungsrate des Differenzdrucks innerhalb der zweiten voreingestellten Dauer 0 ist, oder die Änderungsrate des Differenzdrucks innerhalb der zweiten voreingestellten Dauer größer als oder gleich einem ersten Änderungsratenschwellenwert ist, Bestimmen, dass kein Gasbeutel (13) an der tragbaren Vorrichtung montiert ist; oder
falls die Änderungsrate des Differenzdrucks innerhalb der zweiten voreingestellten Dauer geringer als der erste Änderungsratenschwellenwert ist, Bestimmen, dass der Gasbeutel (13) an der tragbaren Vorrichtung montiert ist.

9. Verfahren nach Anspruch 8, wobei das Erkennen, basierend auf der Änderungsrate des Differenzdrucks innerhalb der zweiten voreingestellten Dauer, der Kennung des an der tragbaren Vorrichtung montierten Gasbeutels (13) umfasst:

als Reaktion auf Informationen, dass die Änderungsrate des Differenzdrucks innerhalb der zweiten voreingestellten Dauer geringer als der erste Änderungsratenschwellenwert ist, Erkennen, basierend auf der Änderungsrate des Differenzdrucks innerhalb der zweiten voreingestellten Dauer und einer Zuordnungsbeziehung zwischen einer Änderungsrate eines Differenzdrucks und einer Kennung eines Gasbeutels (13), der Kennung des an der tragbaren Vorrichtung montierten Gasbeutels (13).

10. Verfahren nach Anspruch 3, wobei die tragbare Vorrichtung umfasst: einen Differenzdrucksensor (24), ein Gasbeutel-Uhrenarmband (12) und mindestens zwei Gasbeutel (13), wobei der Differenzdrucksensor (24) konfiguriert ist, um einen Differenzdruck an einer Luftdüse (221) einer Luftpumpe (22) in der tragbaren Vorrichtung zu erkennen, der Gasbeutel (13) an dem Gasbeutel-Uhrenarmband (12) montiert ist, wenn das Gasbeutel-Uhrenarmband (12), an dem der Gasbeutel (13) montiert ist, an der tragbaren Vorrichtung montiert ist, ein Lufteinlass an dem Gasbeutel (13) mit der Luftdüse (221) in Verbindung steht, und Magnetpole, die an mindestens zwei der Folgenden montiert sind, gleich sind: einem ersten Gasbeutel (13) in den mindestens zwei Gasbeuteln, einem zweiten Gasbeutel (13) in den mindestens zwei Gasbeuteln und einem gasbeutellosen Uhrenarmband (12); das Erkennen, ob der Gasbeutel (13) an der tragbaren Vorrichtung montiert ist, umfasst:

Steuern der Luftpumpe (22), um Luft mit einer voreingestellten Rate zu blasen;

Erhalten einer Änderungsrate des Differenzdrucks innerhalb einer zweiten voreingestellten Dauer basierend auf dem Differenzdruck an der Luftdüse (221), der durch den Differenzdrucksensor (24) erfasst wird; und

Erkennen, basierend auf dem Pegel, der über den Signalausgangs-Pin (213) ausgegeben wird, und der Änderungsrate des Differenzdrucks innerhalb der zweiten voreingestellten Dauer, ob der Gasbeutel (13) an der tragbaren Vorrichtung montiert ist; und

das Erkennen einer Kennung des an der tragbaren Vorrichtung montierten Gasbeutels (13) umfasst:

Erkennen, basierend auf dem Pin, der über den Signalausgangs-Pin (213) den Pegel ausgibt, der größer als der zweite Schwellenwert ist, und der Änderungsrate des Differenzdrucks innerhalb der zweiten voreingestellten Dauer, der Kennung des an der tragbaren Vorrichtung montierten Gasbeutels (13).

**Revendications**

1. Procédé de détection de poche de gaz (13) mis en œuvre par ordinateur pour un dispositif pouvant être porté, comprenant :

le fait de détecter si la poche de gaz (13) est montée sur le dispositif pouvant être porté ;
lorsqu'une poche de gaz (13) est montée sur le dispositif pouvant être porté, le fait de détecter (S401), sur la base d'une circonférence de poignet d'un utilisateur, si la poche de gaz (13) montée sur le dispositif pouvant être porté correspond à la circonférence de poignet de l'utilisateur, comprenant :

en réponse au fait de détecter que la poche de gaz (13) est montée sur le dispositif pouvant être porté, la détection d'un identifiant de la poche de gaz (13) montée sur le dispositif pouvant être porté ; et
le fait de détecter, sur la base d'une relation de mise en correspondance entre une circonférence de poignet et un identifiant d'une poche de gaz (13), si l'identifiant de la poche de gaz (13) montée sur le dispositif pouvant être porté est un identifiant d'une poche de gaz (13) mis en correspondance avec la circonférence de poignet de l'utilisateur ; et
si la poche de gaz (13) montée sur le dispositif pouvant être porté ne correspond pas à la circonférence de poignet de l'utilisateur, l'émission (S402) de premières informations d'invite, dans lequel les premières informations d'invite indiquent de monter une poche de gaz (13) qui correspond à la circonférence de poignet de l'utilisateur;
dans lequel le dispositif pouvant être porté comprend un capteur à effet Hall (21), une pompe à air (22) et une unité de microcontrôleur, MCU, (23), la MCU (23) étant connectée séparément au capteur à effet Hall (21) et à la pompe à air (22) ;
dans lequel le capteur à effet Hall (21) comprend une broche positive (211), une broche négative (212) et une broche de sortie de signal (213) ;
dans lequel la pompe à air (22) est disposée dans le dispositif pouvant être porté, et
une buse d'air (221) reliée à la pompe à air (22) est disposée sur la surface externe du dispositif pouvant être porté ;
dans lequel une entrée d'air est disposée sur la poche de gaz (13), et lorsque la poche de gaz (13) est montée sur le dispositif pouvant être porté, la buse d'air (221) est configurée pour communiquer avec l'entrée d'air de la poche de gaz (13).

2. Procédé selon la revendication 1, dans lequel le fait de détecter, sur la base d'une relation de mise en correspondance entre une circonférence de poignet et un identifiant d'une poche de gaz (13), si l'identifiant de la poche de gaz (13) montée sur le dispositif pouvant être porté est un identifiant d'une poche de gaz (13) mis en correspondance avec la circonférence de poignet de l'utilisateur comprend :

si l'identifiant de la poche de gaz (13) montée sur le dispositif pouvant être porté n'est pas l'identifiant de la poche de gaz (13) mis en correspondance avec la circonférence de poignet de l'utilisateur, le fait de déterminer que la poche de gaz (13) montée sur le dispositif pouvant être porté ne correspond pas à la circonférence de poignet de l'utilisateur ; ou
si l'identifiant de la poche de gaz (13) montée sur le dispositif pouvant être porté est l'identifiant de la poche de gaz (13) mis en correspondance avec la circonférence de poignet de l'utilisateur, le fait de déterminer que la poche de gaz (13) montée sur le dispositif pouvant être porté correspond à la circonférence de poignet de l'utilisateur.

3. Procédé selon la revendication 1 ou 2, dans lequel :

en réponse à la détection d'un pôle magnétique positif, la broche positive (211) émet, par le biais de la broche de sortie de signal (213), un niveau supérieur à un second seuil ;
en réponse à la détection d'un pôle magnétique négatif, la broche négative (212) émet, par le biais de la broche de sortie de signal (213), le niveau supérieur au second seuil ;
lorsque la broche positive (211) ne détecte pas le pôle magnétique positif, la broche positive (211) émet, par le biais de la broche de sortie de signal (213), un niveau inférieur à un premier seuil, dans lequel le second seuil est supérieur au premier seuil ; et
lorsque la broche négative (212) ne détecte pas le pôle magnétique négatif, la broche négative (212) émet, par le biais de la broche de sortie de signal (213), le niveau inférieur au premier seuil.

4. Procédé selon la revendication 3, dans lequel le dispositif pouvant être porté comprend au moins deux poches de gaz, des pôles magnétiques sont disposés sur les poches de gaz des au moins deux poches de gaz, et un pôle magnétique différent est disposé sur chaque poche de gaz (13) ;
le fait de détecter si la poche de gaz (13) est montée sur le dispositif pouvant être porté comprend :

le fait de détecter, sur la base du niveau émis par le biais de la broche de sortie de signal (213), si la poche de gaz (13) est montée sur le dispositif pouvant être porté ; et
la détection d'un identifiant de la poche de gaz (13) montée sur le dispositif pouvant être porté comprend :
la détection, sur la base de la broche qui émet, par le biais de la broche de sortie de signal (213), le niveau supérieur au second seuil, de l'identifiant de la poche de gaz (13) montée sur le dispositif pouvant être porté.

5. Procédé selon la revendication 4, dans lequel les au moins deux poches de gaz comprennent une première poche de gaz (13) et une seconde poche de gaz (13), le pôle magnétique négatif est disposé sur la première poche de gaz (13), le pôle magnétique positif est disposé sur la seconde poche de gaz (13), et le fait de détecter, sur la base du niveau émis par la broche de sortie de signal (213), si la poche de gaz (13) est montée sur le dispositif pouvant être porté comprend :

si le niveau émis par le biais de la broche de sortie de signal (213) est inférieur au premier seuil, le fait de déterminer qu'aucune poche de gaz (13) n'est montée sur le dispositif pouvant être porté ; ou
si le niveau émis par le biais de la broche de sortie de signal (213) est supérieur au second seuil, le fait de déterminer que la poche de gaz (13) est montée sur le dispositif pouvant être porté.

6. Procédé selon la revendication 5, dans lequel la détection, sur la base de la broche qui émet, par le biais de la broche de sortie de signal (213), le niveau supérieur au second seuil, de l'identifiant de la poche de gaz (13) montée sur le dispositif pouvant être porté comprend :

si la broche positive (211) émet, par le biais de la broche de sortie de signal (213), le niveau supérieur au second seuil, le fait de déterminer que la poche de gaz (13) montée sur le dispositif pouvant être porté est la seconde poche de gaz (13) ; ou
si la broche négative (212) émet, par le biais de la broche de sortie de signal (213), un niveau supérieur au second seuil, le fait de déterminer que la poche de gaz (13) montée sur le dispositif pouvant être porté est la première poche de gaz (13).

7. Procédé selon l'une quelconque des revendications 1 à 3, dans lequel le dispositif pouvant être porté comprend un capteur de pression différentielle (24) et un bracelet de montre à poche de gaz (12), le capteur de pression différentielle (24) est configuré pour détecter une pression différentielle au niveau de la buse d'air (221) de la pompe à air (22) dans le dispositif pouvant être porté, la poche de gaz (13) est montée sur le bracelet de montre à poche de gaz (12), et lorsque le bracelet de montre à poche de gaz (12) sur lequel la poche de gaz (13) est montée est monté sur le dispositif pouvant être porté, une entrée d'air sur la poche de gaz (13) communique avec la buse d'air (221) ;
le fait de détecter si la poche de gaz (13) est montée sur le dispositif pouvant être porté comprend :

la commande de la pompe à air (22) pour souffler de l'air à un débit prédéfini ;
l'obtention d'un taux de variation de la pression différentielle au cours d'une seconde durée prédéfinie sur la base de la pression différentielle au niveau de la buse d'air (221) collectée par le capteur de pression différentielle (24) ; et
le fait de détecter, sur la base du taux de variation de la pression différentielle au cours de la seconde durée prédéfinie, si la poche de gaz (13) est montée sur le dispositif pouvant être porté ; et
la détection d'un identifiant de la poche de gaz (13) montée sur le dispositif pouvant être porté comprend :

la détection, sur la base du taux de variation de la pression différentielle au cours de la seconde durée prédéfinie, de l'identifiant de la poche de gaz (13) montée sur le dispositif pouvant être porté.

8. Procédé selon la revendication 7, dans lequel le dispositif pouvant être porté comprend en outre un bracelet de montre sans poche de gaz (12), et lorsque le bracelet de montre sans poche de gaz (12) est monté sur le dispositif pouvant être porté, le bracelet de montre sans poche de gaz (12) bloque la buse d'air (221) de la pompe à air (22) ; et le fait de détecter, sur la base du taux de variation de la pression différentielle au cours de la seconde durée prédéfinie, si le poche de gaz (13) est montée sur le dispositif pouvant être porté comprend :

si le taux de variation de la pression différentielle au cours de la seconde durée prédéfinie est 0, ou si le taux de variation de la pression différentielle au cours de la seconde durée prédéfinie est supérieur ou égal à un premier seuil de taux de variation, le fait de déterminer qu'aucune poche de gaz (13) n'est montée sur le dispositif pouvant être porté ; ou si le taux de variation de la pression différentielle au cours de la seconde durée prédéfinie est inférieur au premier seuil de taux de variation, le fait de déterminer que la poche de gaz (13) est montée sur le dispositif pouvant être porté.

9. Procédé selon la revendication 8, dans lequel la détection, sur la base du taux de variation de la pression différentielle au cours de la seconde durée prédéfinie, de l'identifiant de la poche de gaz (13) montée sur le dispositif pouvant être porté comprend :
en réponse à des informations selon lesquelles le taux de variation de la pression différentielle au cours de la seconde durée prédéfinie est inférieur au premier seuil de taux de variation, la détection, sur la base du taux de variation de la pression différentielle au cours de la seconde durée prédéfinie et d'une relation de mise en correspondance entre un taux de variation d'une pression différentielle et un identifiant d'une poche de gaz (13), de l'identifiant de la poche de gaz (13) montée sur le dispositif pouvant être porté.

10. Procédé selon la revendication 3, dans lequel le dispositif pouvant être porté comprend : un capteur de pression différentielle (24), un bracelet de montre à poche de gaz (12), et au moins deux poches de gaz (13), le capteur de pression différentielle (24) est configuré pour détecter une pression différentielle au niveau d'une buse d'air (221) d'une pompe à air (22) dans le dispositif pouvant être porté, la poche de gaz (13) est montée sur le bracelet de montre à poche de gaz (12), lorsque le bracelet de montre à poche de gaz (12) sur lequel la poche de gaz (13) est montée est monté sur le dispositif pouvant être porté, une entrée d'air sur la poche de gaz (13) communique avec la buse d'air (221), et des pôles magnétiques disposés sur au moins deux parmi les éléments suivants sont identiques : une première poche de gaz (13) dans les au moins deux poches de gaz, une seconde poche de gaz (13) dans les au moins deux poches de gaz, et un bracelet de montre sans poche de gaz (12) ; le fait de détecter si la poche de gaz (13) est montée sur le dispositif pouvant être porté comprend :

la commande de la pompe à air (22) pour souffler de l'air à un débit prédéfini ; l'obtention d'un taux de variation de la pression différentielle au cours d'une seconde durée prédéfinie sur la base de la pression différentielle au niveau de la buse d'air (221) collectée par le capteur de pression différentielle (24) ; et le fait de détecter, sur la base du niveau émis par le biais de la broche de sortie de signal (213) et du taux de variation de la pression différentielle au cours de la seconde durée prédéfinie, si la poche de gaz (13) est montée sur le dispositif pouvant être porté ; et la détection d'un identifiant de la poche de gaz (13) montée sur le dispositif pouvant être porté comprend : la détection, sur la base de la broche qui émet, par le biais de la broche de sortie de signal (213), le niveau supérieur au second seuil et le taux de variation de la pression différentielle au cours de la seconde durée prédéfinie, de l'identifiant de la poche de gaz (13) montée sur le dispositif pouvant être porté.

FIG. 1

Hall effect sensor **21**

MCU **23**

Positive pin 211

Negative pin 212

Signal output pin 213

Air pump **22**

Inflate

Air nozzle **221**

Magnetic pole

Gasbag **13**

Wearable device

a

Wearable device

Gasbag detachment component

Positive pin 211

Negative pin 212

Air nozzle **221**

Magnetic pole Gasbag **13**

b

FIG. 2

Output a low level

| Positive pin | Negative pin | Air nozzle |

Non-gasbag watchband

a

Output a high level

| Positive pin | Negative pin | Air nozzle |

Negative pole

First gasbag

b

Output a high level

| Positive pin | Negative pin | Air nozzle |

Positive pole

Second gasbag

c

FIG. 3

Detect, based on a wrist circumference of a user, whether a gasbag mounted on a wearable device matches the wrist circumference of the user

S401

No

Yes

Output first prompt information, where the first prompt information is used to prompt the user to mount a gasbag that matches the wrist circumference of the user

S402

Control the gasbag to be inflated, to detect a blood pressure of the user

S403

FIG. 4

FIG. 5

51

Input a wrist circumference

| a | cm |

511

512

Start to measure

a

The current gasbag does not match the wrist circumference. Please replace the gasbag with a second gasbag that matches the wrist circumference

513

Start to measure

b

FIG. 6

FIG. 7

Perform magnetic pole
detection by using a
Hall effect sensor
S801

Determine, in
response to detecting
that a positive pin and
a negative pin on the
Hall effect sensor
output a low level,
that no gasbag is
mounted on a
wearable device
S802

In response to detecting
that a positive pin on
the Hall effect sensor
outputs a high level,
control a gasbag to be
inflated, and obtain a
change rate of a
differential pressure
within second preset
duration
S804

Determine, in
response to detecting
that a negative pin on
the Hall effect sensor
outputs a high level,
that a gasbag
mounted on a
wearable device is a
first gasbag
S803

Determine, based on
the change rate of the
differential pressure
within the second
preset duration, that the
gasbag mounted on a
wearable device is a
second gasbag or a
non-gasbag watchband
S805

Detect, based on a wrist
circumference of a user,
whether the gasbag
mounted on the wearable
device matches the wrist
circumference of the user
S401

No                                    Yes

Output first prompt
information, where the
first prompt information
is used to prompt the user
to mount a gasbag that
matches the wrist
circumference of the user
S402

Control the gasbag to
be inflated, to detect a
blood pressure of the
user
S403

FIG. 8

900

901 902 903

| Detection module | Output module | Sending and receiving module |

FIG. 9

**EP 4 388 992 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2020085319 A1
- CN 212213721 U
- CN 108403102 A
- US 2021251500 A1

### Non-patent literature cited in the description

- **DARBAR RAJKUMAR et al.** *Using Hall Effect Sensors for 3D Space Text Entry on Smartwatches*